# EUROPEAN PATENT SPECIFICATION

(11) **EP 1 849 411 B1**
(45) Date of publication and mention of the grant of the patent: **25.12.2019**
(21) Application number: 07015964.5
(22) Date of filing: 17.06.2005
(51) Int. Cl.: A61B 5/107, A61B 5/00, A61C 13/00, G01J 3/50, A61C 19/04

(54) **Method for providing data associated with the intraoral cavity**
Verfahren zum Bereitstellen von Daten im Zusammenhang mit der Mundhöhle
Méthode pour fournir des données concernant la cavité intra-orale

(30) Priority: 17.06.2004 US 580109 P; 17.06.2004 US 580108 P
(43) Date of publication of application: 31.10.2007
(62) Divisional of application: 05013110.1
(73) Proprietor: Align Technology, Inc., San Jose, CA 95134 (US)
(72) Inventor: Babayoff, Noam, Rishon le Zion 75670 (IL)
(74) Representative: Grünecker Patent- und Rechtsanwälte PartG mbB

(56) References cited:
- EP-A- 0 360 657
- EP-A- 1 252 867
- WO-A-2004/008981
- DE-A1- 19 922 870
- JP-A- 2000 293 689
- US-A- 5 372 502
- US-A- 5 417 572
- US-A- 5 743 730
- US-A- 6 118 521
- US-A1- 2001 002 310
- US-A1- 2003 198 384
- US-A1- 2004 029 068

## Description

### FIELD OF THE INVENTION

The present invention relates to colour three-dimensional numerical entities representative of tissues, particularly of the intraoral cavity. In particular, the present invention is concerned with the manipulation of such entities to provide data that is useful in procedures associated with the oral cavity, specially dental surfaces thereof.

### BACKGROUND OF THE INVENTION

There are many procedures associated with the oral cavity in which a precise three-dimensional representation of the cavity is very useful to the dental practitioner. Herein, "practitioner" refers to any one of a dentist, dental surgeon, dental technician, orthodontist, prosthodontist, or any other caregiver that may be involved in determining, preparing or providing dental treatment to a patient, particularly orthodontic treatment.

Such representations enable the practitioner to study the cavity of individual patients in a similar manner to the study of the traditional plaster model. More importantly, three-dimensional numerical entities of the dental cavity also allow the practitioner to study alternative methods or approaches when dealing with particular dental problems of any given patient. For example, in orthodontics, a computer model of a patient's teeth may be manipulated to arrive at the optimal arrangement for brackets to ensure effective treatment of crooked teeth. For such procedures, it is often useful to provide a three-dimensional representation of the individual teeth, each of which can be moved independently in a computer simulation of the orthodontic treatment plan and orthodontic record. Hitherto, identification and separation of the data sets representative of the individual teeth has been performed manually.

In US 6,739,869, assigned to the present Assignee, a method for virtual orthodontic treatment is disclosed in which a virtual set of orthodontic components is associated, in a virtual space, with a first virtual three-dimensional image of teeth, and then by a set of rules which define the effect of the set of components' teeth, the effect of the virtual treatment can be computed. This virtual treatment can be used to predict the results of a real-life orthodontic treatment as to design such a treatment.

Another procedure relates to the manufacture of a dental prosthesis, such as a crown or bridge, which requires the finish line, or transition boundary between the prosthesis and the dental preparation to be precisely defined in three-dimensions. Obtaining the finish line coordinates from a computer model is more efficient and often more accurate than from a plaster cast, and moreover facilitates the production of such a prosthesis, for example via CNC machining, rapid prototyping, or other computerised technologies, if desired.

There are also procedures in which particularly good three-dimensional definition of a large area of the intraoral cavity is required, and it may be necessary at times to scan parts of the cavity sequentially and then "stitch" the various data sets together. Thus, surface topologies of adjacent portions, at times from two or more different angular locations relative to the structure, are determined and the topologies are combined, in a manner known per se. In practice, such stitching is usually performed automatically, by identifying a surface profile or topography corresponding to at least part of the data of one data set that is substantially identical to that of another data set. The data sets are then manipulated such as to match the coordinates of the surface profile between the data sets to obtain a larger numerical entity comprising these data sets in their proper spatial relationship. However, the data sets often include surface data relating to the soft tissues such as gums, cheeks and tongue, for example, which may distort and move while the different scans are performed. This relative movement makes the stitching procedure more problematic, since parts of the data sets thus obtained will never synchronise, even though they relate to overlapping portions of the intra-oral cavity.

In procedures relating to the manufacture of a dental prosthesis such as a crown or bridge, or in other dental restorations, it is important to match the colour and texture of the prosthesis with that of the surrounding teeth in the vicinity of the target area in which the prosthesis is to be implanted, to give the prosthesis or restoration a natural appearance. Traditional methods of colour matching are based on visual comparison between a removable tooth-shaped colour tab of a shade guide, such as for example Vita, and the surrounding teeth. The practitioner can then choose the standard shade that best matches the overall colour of the other teeth. The appropriate shade reference can then be communicated to the laboratory that is to manufacture the prosthesis. Similar matching methods are routinely employed for enabling the colour of filler material to be matched to the tooth that requires a filling. It should be noted that unlike the tooth shades from the shade guides, which are uniform in colour, a tooth have many different shades. For example, tooth stains and the like alter then tooth's colour locally. Attempts have been made at providing a more exact match. For example in US 5,800,164 a system is described for the selection of form and colour structure of teeth, and includes several assortments of models and representations as well as layering diagrams of different tooth forms and colour structures. A comparison of form and colour structure between the patient's teeth and the models is made, and a suitable assortment is selected. The layering diagrams thus produced enable the production of the prosthesis to be carefully controlled to provide the desired form and colour structure. In US 4,836,674 a method and apparatus are described for obtaining the best colour match with respect to adjacent teeth, for different lighting conditions, but considers only the overall colour of the teeth, and does not address local variations of colour in the teeth. In US 6,525,819, a colourimeter is provided for providing, inter alia, the one-dimensional array of colour data along a line on the surface of the intra-oral cavity, including the teeth. In US 5,766,006 a method is described for comparing the tooth shade after the patient's teeth are whitened, in which a colour two-dimensional image of the tooth is obtained before the whitening, comparing the colour information representative of the colour of the tooth, and identifying one or more tooth shades with a combined colour corresponding to the colour of the tooth. After whitening the tooth, another image is taken thereof and compared to the image before whitening.

Other US patents of general background interest include the following.

In US 5,851,113, US 5,871,351, US 6,118,521, US 6,239,868, US 6,246,479, US 6,417,917, US 6,538,726, US 6,570,654 and US 6,573,984 colour measuring systems and methods such as for determining the colour or other characteristics of teeth are disclosed, in which these characteristics are obtained using a fibre optics arrangement which is targeted onto a specific area of each tooth and at a certain height and angle with respect to this area. The colour measurement data obtained may be used to implement processes for forming dental prostheses.

In US 6,007,332, a method and system for determining the colour characteristic of a tooth employs the photographic imaging of the tooth, and the photographing of visually selected colour standards, to achieve the final selection of the closest colour match. The resulting photographic images, which may be on a single photograph, are subjected to calorimetric or spectrophotometric analysis to achieve the final selection of the closest match.

In US 6,030,209, a spectrophotometer device measures and provides general colourimetric data regarding hue, chroma, value, and translucency for each of the incisal, middle, and cervical regions of the tooth. This colourimetric data is then converted via an algorithm to a recipe that the dentist or dental technician follows in constructing a dental restoration. A porcelain system is also provided which allows a user to independently alter one colour component while not affecting the other three colour components.

In US 6,033,222, a fabrication method for dental translucent restorations involves forming a dental die from impression of tooth stump, in which a series of die spacers of differing shades are used to match or replicate an assortment of tooth stump or dentin tooth shades. The method comprises applying colour and shade to match dentist's prescription, forming a dental wax-up and then the dental structure.

In US 6,190,170, and US 6,238,567, an automated tooth shade analysis and matching method is used for making a dental prosthesis. An image of the teeth is acquired including black and white normalization references for determining absolute black and absolute white within the image. The image is normalized in accordance with the normalization references, and then standardized by matching the pixels of the normalized image to selected shade standards for the prosthesis.

In US 6,379,593, a method for producing a multi-coloured dental restoration is disclosed. A compacting die is filled with a matrix of two or more plastics materials of different colours, and the pressure is applied to the matrix in the die.

The 3D surface structure of the teeth is not considered in any of the aforesaid patents.

A method for defining a finish line of a dental prosthesis is known from WO 2004/008981.

A method for segmenting an image is known from US 2003/0198384.

A method and system for integrated orthodontic treatment planning using a unified workstation is known from US 2004/0029068

### SUMMARY OF THE INVENTION

"Colour" is used herein to refer to a perceived optical characteristic, including one or more of the following: hue, chroma, value, translucency, reflectance.

"Hue" is used herein to refer to a colour or to the name of a colour, for example primary or other colours such as red, green, blue, violet, green and so on, or to combination of colours, such as for example yellowish green. The hues of primary interest herein include red, and shades of white including yellow for intraoral cavity tissues, and other hues representative of the colour of filings and so on.

"Chroma" is used herein to refer to strength, intensity or saturation of the hue.

"Value" is used herein to refer to the brightness of a colour.

"Translucency" is used herein to refer to quality of transmitting and diffusing light, generally ranging from opaque to transparent.

"Reflectance" is used herein to refer to the quality of reflecting light incident on the tooth.

"Numerical entity" is used herein to mean a data set or to a set of instructions that is manipulable by automated numerical processing means, such as a computer for example, particularly such that specific data may be obtained therefrom.

Such manipulation may be under manual, interactive, partial or fully automated control.

The invention provides a method for providing a finish line for a preparation area in an oral cavity according to claim 1. Further aspects are mentioned in the dependent claims.

An embodiment is particularly useful for providing a finish line for a preparation area in said intraoral cavity, though it may also be used for virtually separating the teeth from the gums. The method comprises:
(a) comparing the colour data for each pair of spatially adjacent data points in said numerical entity;
(b) if the colour data for said pair of data points are substantially different one from the other, providing one said data point of said pair of data points;
   and
(c) applying a search algorithm for identifying said finish line in said numerical entity, wherein said application of said algorithm is initially applied to a part of said entity corresponding to the said provided data points of step (b).

Optionally, in step (b) the data point of said pair of data points having colour data associated with a hard tissue is provided for step (c).

Typically, step (a) comprises:
separating said surface geometry and colour data into at least two tissue data sets, wherein
a first said tissue data set comprises surface geometry and colour data, wherein said colour data thereof is correlated with a colour representative of said first tissue; and
a second said tissue data set comprises surface geometry and colour data wherein said colour data thereof is correlated with a colour representative of said second tissue.

The first tissue comprises hard tissues such as teeth, and the soft tissue comprises at least one of gums, tongue, cheeks and lips.

The first tissue data set may correspond to a plurality of teeth of said intraoral cavity, and in the next step the first tissue data set is divided into a plurality of sub data sets, wherein each said sub data set correspond to a different said tooth.

In another aspect of the present invention, a computer readable medium according to claim 9 is provided.

The medium may comprise, for example, optical discs, magnetic discs, magnetic tapes, and so on.

### BRIEF DESCRIPTION OF THE DRAWINGS

In order to understand the invention and to see how it may be carried out in practice, a preferred embodiment will now be described, by way of nonlimiting example only, with reference to the accompanying drawings, in which:
**Fig. 1** illustrates the main elements of embodiments of devices for creating a three-dimensional colour numerical entity that is manipulated according to the present invention.
**Figs. 2A, 2B****,** **2C** graphically illustrates the creation of a three dimensional colour entity from a three dimensional monochrome entity and a two dimensional colour entity.
**Fig. 3** graphically illustrates an alignment procedure according to the invention for aligning the X-Y coordinates of a three dimensional monochrome entity with corresponding coordinates of a two dimensional colour entity.
**Figs. 4A** **and** **4B** schematically illustrate the main elements of a portion of a device used for providing a three dimensional monochrome entity.
**Figs. 5A, 5B, 5C** illustrate in plan view, side view and isometric view, respectively, a probe used in first embodiment of the device of Fig. 1 to provide a two dimensional colour entity.
**Fig. 6** illustrates in side view a sheath for a probe used in second embodiment of the device of Fig. 1 to provide a two dimensional colour entity.
**Fig. 7A** illustrates in side view a probe used in third embodiment of the device of Fig. 1 to provide a two dimensional colour entity. **Fig. 7B** illustrates the transmission and reflection characteristics of a typical dichroic coating used in the probe of Fig. 7A.
**Fig. 8** illustrates in side view the general arrangement of the main elements used in fourth embodiment of the device of Fig. 1 to provide a two dimensional colour entity.
**Fig. 9** illustrates an LED arrangement used with the embodiment of Fig. 8.
**Fig. 10** illustrates an alternative illumination arrangement used with the embodiment of Figure 8. Fig. 10A illustrates details of the tri-colour disc used with the illumination arrangement of Fig. 10.
**Fig. 11** illustrates in side view the general arrangement of the main elements used in fifth embodiment of the device of Fig. 1 to provide a two dimensional colour entity.
**Fig. 12** illustrates in side view the general arrangement of the main elements used in sixth embodiment of the device of Fig. 1 to provide a two dimensional colour entity,
**Fig. 13** illustrates in side view the general arrangement of the main elements used in seventh embodiment of the device of Fig. 1 to provide a two dimensional colour entity.
**Fig. 14** illustrates the main steps of a method for differentiating one tissue from another tissue.
**Fig. 15** illustrates the main steps of a method for stitching data sets of overlapping areas.
**Fig. 16** illustrates the main steps of a method for automatically defining the finish line of a preparation.
**Fig. 17** illustrates the main steps of a method for shading of a prosthesis.
**Fig. 18** illustrates a portion of the intraoral cavity on which it is desired to implant a dental prosthesis.
**Fig. 19** schematically illustrates a transformation step according to the method of Fig. 17.
**Fig. 20** is a block diagram of a system useful for implementing the present invention.

### DETAILED DESCRIPTION OF THE INVENTION

The first step of the method according to the present invention relates to providing at least one numerical entity that is representative of the three-dimensional surface geometry and colour of at least part of the intra-oral cavity.

The said numerical entity is typically at least "four-dimensional", that is, each data point of the data set comprises at least four prime independent variables. In the preferred embodiments of the invention, three of the prime independent variables relate to spatial coordinates of a surface, typically defined along orthogonal Cartesian axes, x, y, z. Alternatively, these variables may be defined along polar axes or any other geometric system in which a surface may be described. The fourth prime independent variable refers to a colour parameter that is expressed numerically and associated with the spatial coordinates. The colour parameter may itself be comprised of independent prime colour variables - for example relating to the red, blue and green (RGB) components associated with the colour parameter. Alternatively, the colour parameter may be expressed in terms of the Hue, Saturation and Intensity (HIS). Alternatively, any other colour parameter may be used, including parameters that provide a measure of internal reflectance and translucency, or any other optical property of teeth.

Thus, the numerical entity may comprise a data set of a plurality of 4-dimensional arrays - (x, y, z, c), wherein each array represents the x, y, z, geometrical coordinates and the colour c of a point on a surface within the intra-oral cavity.

Any suitable means may be used to provide the numerical entity. For example, a three-dimensional surface scanner with colour capabilities may be used. Advantageously, such a scanner makes use of confocal imaging for providing an accurate three-dimensional representation of the target surface within the intra-oral cavity. Colour values are then added to each data point of this data set by obtaining a two-dimensional colour image of the target surface, and then mapping the colour values of the two-dimensional image onto the three-dimensional "image".

The following are examples on how to obtain the 3D colour numerical entity.

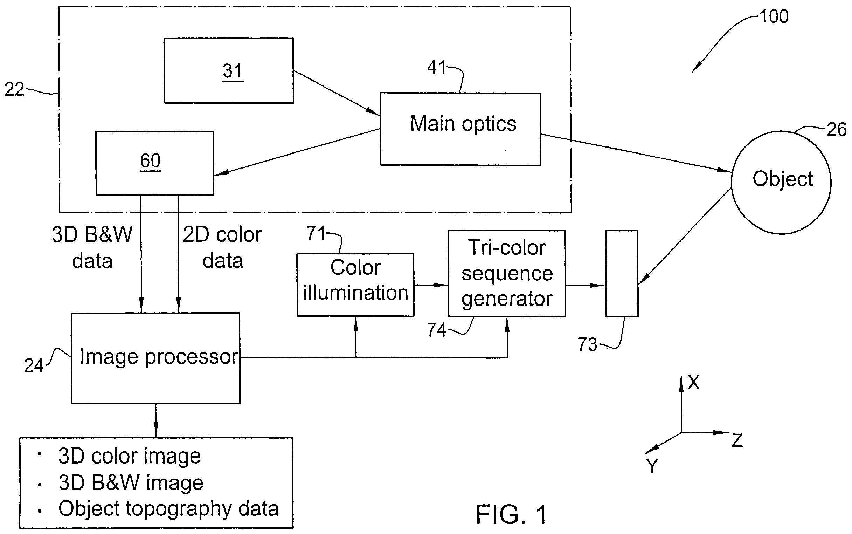

Reference is first being made to Fig. 1 which illustrates the general relationship between the various elements of a device for providing a 3D colour numerical entity, generally designated with the numeral **100**, according to the embodiments of the device described herein.

The device **100** comprises a main illumination source **31** for illuminating the object of interest **26**, typically a part of the intraoral cavity, and is optically coupled to main optics **41** to provide depth **Z** values for an array range of **X-Y** points (according to a known frame of reference) along the surface of the object **26.** Detection optics **60** comprises an image sensor, typically a CCD, that is preferably monochromatic to maximise the resolution of the device, and which typically defines the X-Y frame of reference. Alternatively, the CCD may be adapted to receive colour images. The detection optics **60** receives image data from the main optics **41** and the image processor **24** determines the depth **Z** values for each **X-Y** point illuminated on the object **26** based on this image data. In this manner, a manipulable three-dimensional numerical entity **E** comprising the surface coordinates of the object **26**.

The device **100** further comprises colour illuminating means, such as for example a tri-colour sequence generator **74**, for selectively illuminating the object 26 with suitable colours, typically Green, Red and Blue, and for each such monochromatic illumination, a two dimensional image of the object **26** is captured by the detection optics **60.** The processor **24** then processes the three differently coloured monochromatic images and combines the same to provide a full colour 2D image of the object. The device **100** is configured for providing colour data for an array of X-Y points that is according to the same frame of reference as the X-Y array used for obtaining the 3D entity.

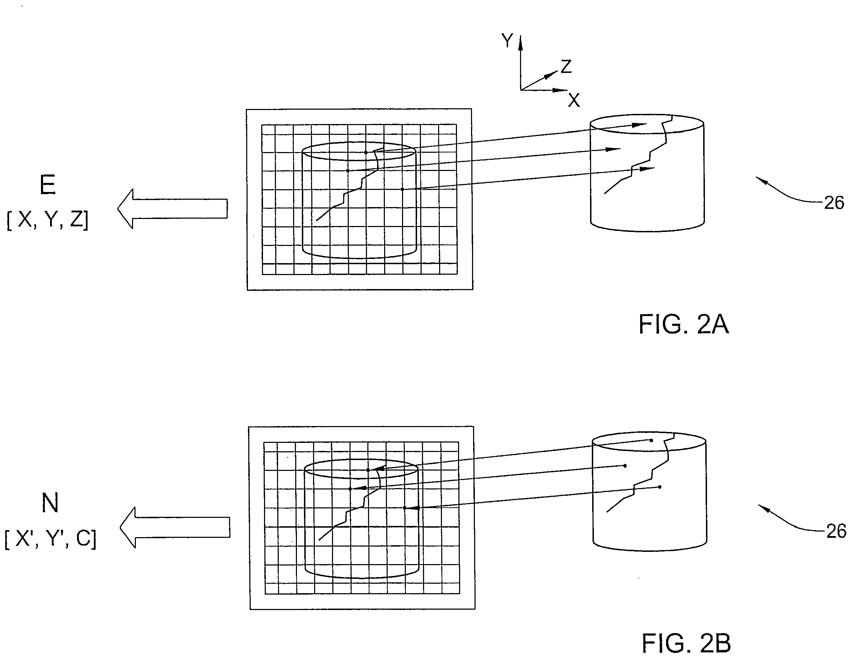

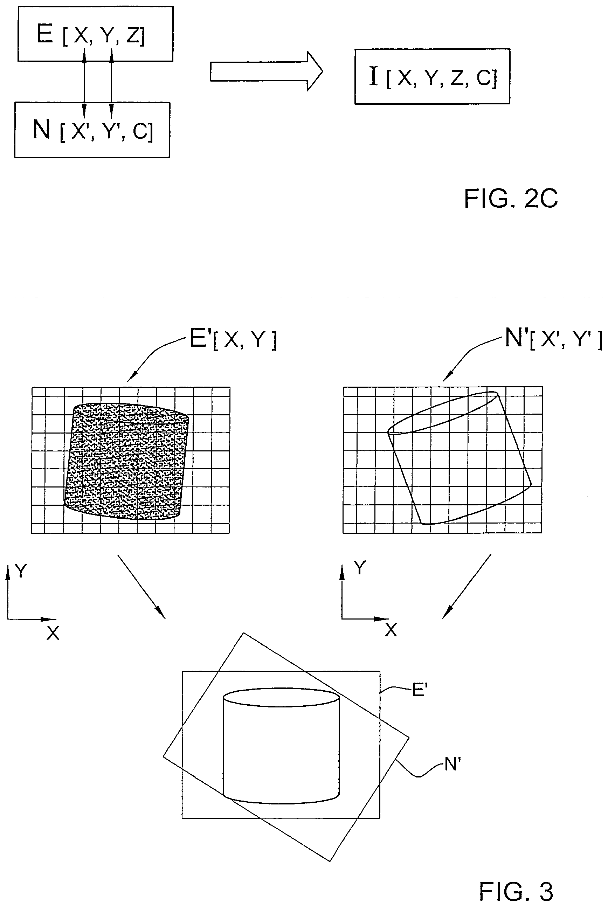

The processor **24** aligns the 2D colour image with the 3D entity previously created, and then provides colour values to this entity by mapping colour values to the entity at aligned **X-Y** points. Such alignment is straightforward because both the 3D data and the 2D colour data are referenced to the same X-Y frame of reference. Referring to Figs. 2A, 2B, 2C, the mapping procedure is performed as follows. A three-dimensional numerical entity **E** is obtained by determining depth **Z**-values for a grid of **X-Y** points, illuminated via main optics **41** and determined by image processor **24.** The entity **E** thus comprises an array of (**X, Y, Z**) points, as illustrated in Fig. 2A. The **X-Y** plane of entity **E** is substantially parallel to the sensing face of the image sensing means of the detection optics **60**, typically a CCD. Almost concurrently, i.e., either just before or just after the readings for determining the 3D entity **E** are obtained by the detection optics **60**, a 2D colour image of the object **26** is taken using the same detection optics **60**, at substantially the same relative spatial disposition between the detection optics **60** and the object **26**, Fig. 2B. If a monochromatic CCD is used, the 2D colour image obtained is a composite created from three separate monochromatic images, each provided by illuminating the object **26** with a different colour, such as for example red, green and blue. The 2D colour image thus corresponds to another entity **N** comprised of the location and colour value of each pixel forming this image, **(X', Y', C).** The **X'-Y'** coordinates of the pixels are on a plane substantially parallel to the **X-Y** plane of the entity **E**, and furthermore these coordinates represent substantially the same part of the object **26** as the **X-Y** coordinates of entity **E.** The reason for this is that the optical information that is used for creating both the 3D entity **E** and the colour 2D entity **N** are obtained almost simultaneously with a very small time interval therebetween, and typically there is insufficient time for any significant relative movement between the image plane of the detection optics **60** and the object **26** to have occurred between the two scans. Thus, similar **X-Y** and **X'-Y'** coordinates in the entities **E** and **N**, respectively, will substantially represent the same part of the object **26.** Accordingly, the colour value **C** of each pixel of entity **N** can be mapped to the data point of entity **E** having **X-Y** coordinates that are the same as the **X'-Y'** coordinates of the pixel, whereby to create another entity **I** comprising surface coordinate and colour data, **(X, Y, Z, C),** as illustrated in Fig. 2C.

Were the relative angle and disposition between the plane of the sensing face of the detection optics **60** with respect to the object **26** change significantly between the 2D and the 3D scans, then the **X-Y** coordinates of entity **E** having similar values to the **X'-Y'** coordinates of entity **N** could correspond to different parts of the object **26**, and thus it may then be difficult to map the colour values of entity **N** to entity **E.** However, if only a small movement between the detection optics **60** with respect to the object **26** occurs, particularly involving a relative translation or a rotation about the depth direction (Z), but substantially no change in the angular disposition between detection optics **60** and the object **26** about the **X** or **Y** axes, it may still be possible to map the colour values of entity **N** to entity **E**, but first an alignment procedure must be followed.

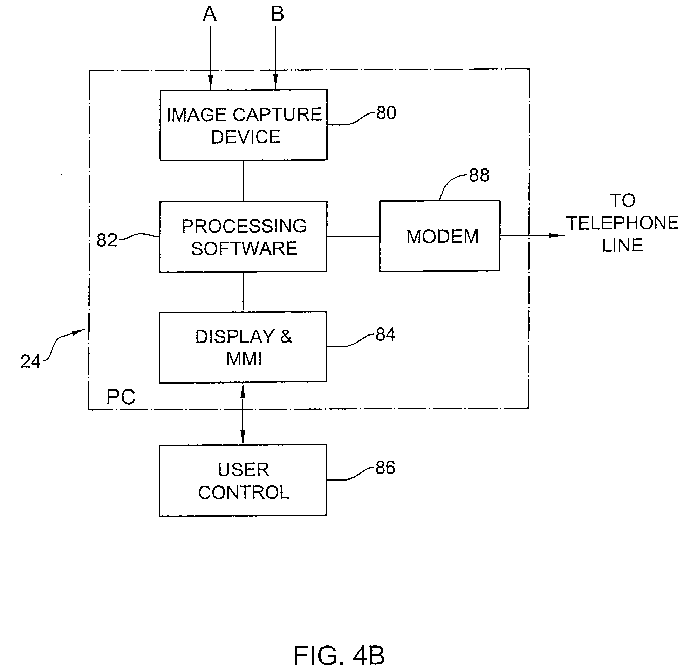

Referring to Fig. 3, such an alignment procedure may be based on optical character recognition (OCR) techniques. In the **X-Y** plane, the **X-Y** coordinates of entity E can be divided up into two groups, one group comprising **Z** values corresponding to the depth of the object, and a second group for which no reasonable **Z** value was found, and this group corresponds to the background relative to object **26.** The profiles of shapes represented by the **X-Y** coordinates of the first group of entity **E**, herein referred to as another entity **E'**, are then optically compared with profiles of shapes corresponding to the **X'-Y'** coordinates of entity **N**, herein referred to as another entity **N'.** Accordingly, entity **E'** is translated or rotated (coplanarly) with respect to entity **N'** until a best fit between the optical shapes between the two entities is obtained, using OCR techniques that are well known in the art. Typically, the image processor, or another computer, will attempt to align the outer border of the object **26** as seen along the **Z**-axis and encoded in entity **E** with optical elements in the 2D colour image encoded in entity **N.** Thereafter, the colour value **C** of each **X'-Y'** coordinate of entity **N** is mapped to the appropriate data point of entity **E** having the aligned **X-Y** coordinates corresponding thereto. The colour mapping operation to create entity **I** may be executed by any suitable microprocessor means, typically processor **24** of the device **100** (Fig. 4B).

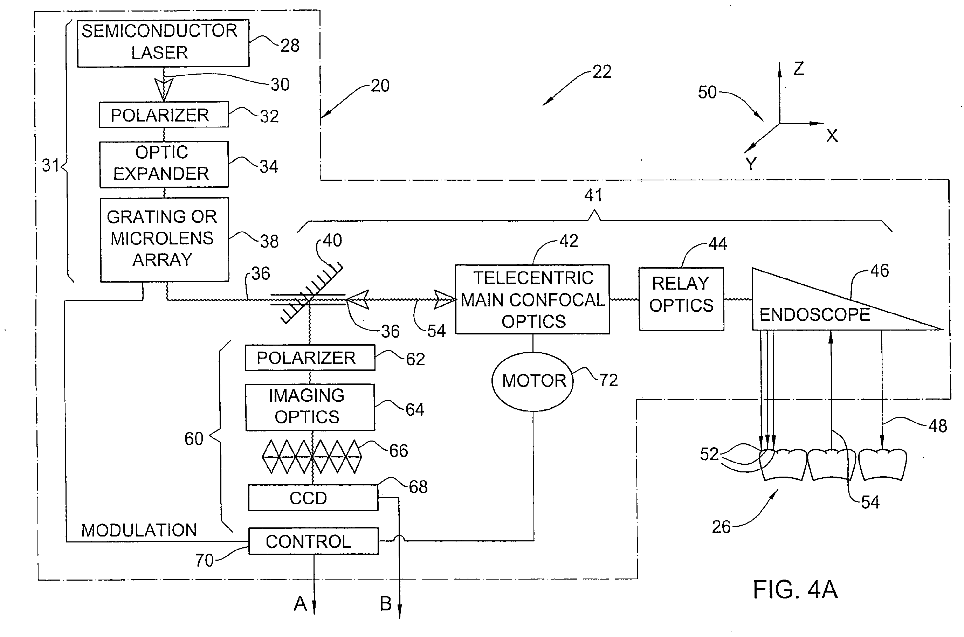

The main optics **41*,*** main illumination source **31**, detection optics **60** and image processor **24** are now described with reference to Figs. 4A and 4B which illustrate, by way of a block diagram an embodiment of a system **20** for confocal imaging of a three dimensional structure according to WO 00/08415. Alternatively, any suitable confocal imaging arrangement may be used in the present device.

The system **20** comprises an optical device **22** coupled to a processor **24.** Optical device **22** comprises, in this specific embodiment, a semiconductor laser unit **28** emitting a laser light, as represented by arrow **30.** The light passes through a polarizer **32** which gives rise to a certain polarization of the light passing through polarizer **32.** The light then enters into an optic expander **34** which improves the numerical aperture of the light beam **30.** The light beam **30** then passes through a module **38**, which may, for example, be a grating or a micro lens array which splits the parent beam **30** into a plurality of incident light beams **36**, represented here, for ease of illustration, by a single line. The operation principles of module **38** are known per se and the art and these principles will thus not be elaborated herein.

The optical device **22** further comprises a partially transparent mirror **40** having a small central aperture. It allows transfer of light from the laser source through the downstream optics, but reflects light traveling in the opposite direction. It should be noted that in principle, rather than a partially transparent mirror other optical components with a similar function may also be used, e.g. a beam splitter. The aperture in the mirror **40** improves the measurement accuracy of the apparatus. As a result of this mirror structure the light beams will yield a light annulus on the illuminated area of the imaged object as long as the area is not in focus; and the annulus will turn into a completely illuminated spot once in focus. This will ensure that a difference between the measured intensity when out-of- and in-focus will be larger. Another advantage of a mirror of this kind, as opposed to a beam splitter, is that in the case of the mirror internal reflections which occur in a beam splitter are avoided, and hence the signal-to-noise ratio improves.

The unit further comprises a confocal optics **42**, typically operating in a telecentric mode, a relay optics **44**, and an endoscopic probing member **46.** Elements **42**, **44** and **46** are generally as known per se. It should however be noted that telecentric confocal optics avoids distance-introduced magnification changes and maintains the same magnification of the image over a wide range of distances in the **Z** direction (the **Z** direction being the direction of beam propagation). The relay optics enables to maintain a certain numerical aperture of the beam's propagation.

The endoscopic probing member **46** typically comprises a rigid, light-transmitting medium, which may be a hollow object defining within it a light transmission path or an object made of a light transmitting material, e.g. a glass body or tube. At its end, the endoscopic probe typically comprises a mirror of the kind ensuring a total internal reflection and which thus directs the incident light beams towards the teeth segment **26.** The endoscope **46** thus emits a plurality of incident light beams **48** impinging on to the surface of the teeth section.

Incident light beams **48** form an array of light beams arranged in an **X-Y** plane, in the Cartesian frame **50**, propagating along the **Z** axis. As the surface on which the incident light beams hits is an uneven surface, the illuminated spots **52** are displaced from one another along the **Z** axis, at different (Xᵢ,Yᵢ) locations. Thus, while a spot at one location may be in focus of the optical element **42**, spots at other locations may be out-of-focus. Therefore, the light intensity of the returned light beams (see below) of the focused spots will be at its peak, while the light intensity at other spots will be off peak. Thus, for each illuminated spot, a plurality of measurements of light intensity are made at different positions along the Z-axis and for each of such (Xᵢ,Yᵢ) location, typically the derivative of the intensity over distance (Z) will be made, the Zᵢ yielding maximum derivative, Z₀, will be the in-focus distance. As pointed out above, where, as a result of use of the punctured mirror **40**, the incident light forms a light disk on the surface when out of focus and a complete light spot only when in focus, the distance derivative will be larger when approaching in-focus position thus increasing accuracy of the measurement.

The light scattered from each of the light spots includes a beam traveling initially in the **Z**-axis along the opposite direction of the optical path traveled by the incident light beams. Each returned light beam **54** corresponds to one of the incident light beams **36.** Given the unsymmetrical properties of mirror **40**, the returned light beams are reflected in the direction of the detection optics **60.** The detection optics **60** comprises a polarizer **62** that has a plane of preferred polarization oriented normal to the plane polarization of polarizer **32.** The returned polarized light beam **54** pass through an imaging optic **64**, typically a lens or a plurality of lenses, and then through a matrix **66** comprising an array of pinholes. CCD camera has a matrix or sensing elements each representing a pixel of the image and each one corresponding to one pinhole in the array **66.**

The CCD camera is connected to the image-capturing module **80** of processor unit **24.** Thus, each light intensity measured in each of the sensing elements of the CCD camera is then grabbed and analyzed, in a manner to be described below, by processor **24.**

Unit **22** further comprises a control module **70** connected to a controlling operation of both semi-conducting laser **28** and a motor **72.** Motor **72** is linked to telecentric confocal optics **42** for changing the relative location of the focal plane of the optics **42** along the Z-axis. In a single sequence of operation, control unit **70** induces motor **72** to displace the optical element **42** to change the focal plane location and then, after receipt of a feedback that the location has changed, control module **70** will induce laser **28** to generate a light pulse. At the same time, it will synchronize image-capturing module **80** to grab data representative of the light intensity from each of the sensing elements. Then in subsequent sequences the focal plane will change in the same manner and the data capturing will continue over a wide focal range of optics **44.**

Image capturing module **80** is connected to a CPU **82**, which then determines the relative intensity in each pixel over the entire range of focal planes of optics **42**, **44.** As explained above, once a certain light spot is in focus, the measured intensity will be maximal. Thus, by determining the Zᵢ corresponding to the maximal light intensity or by determining the maximum displacement derivative of the light intensity, for each pixel, the relative position of each light spot along the **Z**-axis can be determined. Thus, data representative of the three-dimensional pattern of a surface in the teeth segment, can be obtained. This three-dimensional representation may be displayed on a display **84** and manipulated for viewing, e.g. viewing from different angles, zooming-in or out, by the user control module **86** (typically a computer keyboard).

The device **100** further comprises means for providing a 2D colour image of the same object **26**, and any suitable technique may be used for providing the colour image. A number of such techniques are described below.

The first technique is based on illuminating the object **26** sequentially with three different coloured lights such as red, green and blue, and capturing a monochromatic image corresponding to each colour via CCD **68** and the image capture device **80** (see Figs. 4A, 4B). Referring to Fig. 1, tri-colour light sources **71**, i.e., one or more light sources that provide illuminating radiations to the object **26** in a plurality of different colours, are coupled to a tri-colour sequence generator **74**, which are suitably controlled by the processing unit **24** to provide the three coloured illuminations via delivery optics **73** in a predetermined sequence. The coloured illuminations are provided at a relative short time interval, typically in the range of about 0 to **100** milliseconds, I some cases being in the order of 50 milliseconds, with respect to the 3D scan, directly before or after the same. Suitable processing software **82** combines the three images to provide a 2D colour image comprising an array of data points having location **(X,Y)** and colour **(C)** information for each pixel of the 2D colour image.

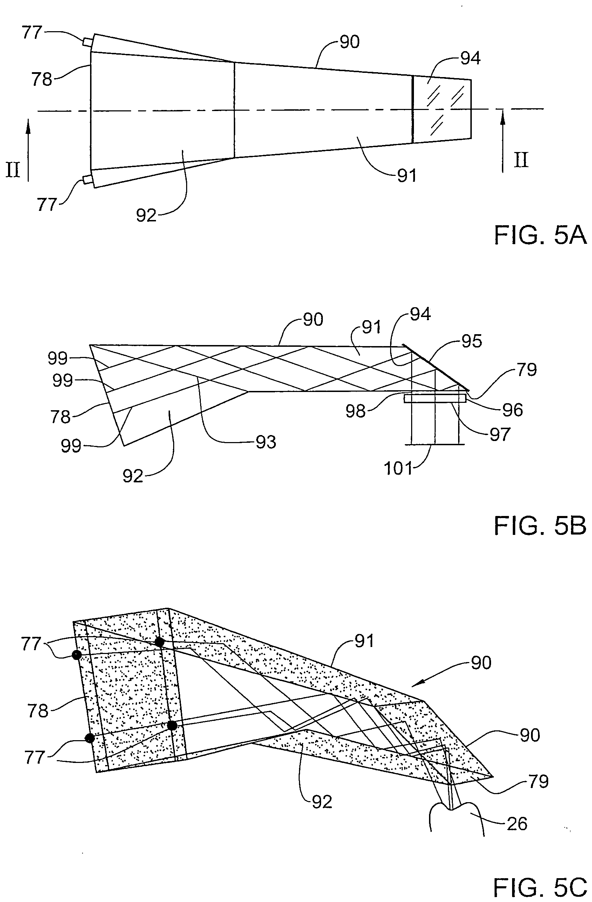

According to a first embodiment of the device **100**, the delivery optics **73** is integral with endoscope **46**, which is in the form of a probing member **90**, as illustrated in Figs. 5A, 5B and 5C. The probing member **90** is made of a light transmissive material, typically glass and is composed of an anterior segment **91** and a posterior segment **92**, tightly glued together in an optically transmissive manner at **93.** Slanted face **94** is covered by a totally reflective mirror layer **95.** Glass disk **96** defining a sensing surface **97** may be disposed at the bottom in a manner leaving an air gap **98.** The disk is fixed in position by a holding structure which is not shown. Three light rays are **99** from the main optics **42** are represented schematically. As can be seen, they bounce at the walls of the probing member at an angle in which the walls are totally reflective and finally bounce on mirror **95** and reflected from there out through the sensing face **97.** The light rays focus on focusing plane 101, the position of which can be changed by the focusing optics (not shown in this figure). The probe member **90** comprises an interface **78** via which optical communication is established with the relay optics **44** and the remainder of the device **100.** The probe **90** further comprises a plurality of tri-colour LED's **77**, for providing the coloured illumination to the object **26.**

The LED's 77 typically comprise different LED's for providing blue radiation and green radiation when red illuminating radiation is used as the illumination source 31 for the main optics 41 when creating the 3D entity. Alternatively, if a blue illuminating radiation is used as the illumination source 31, the LED's 77 may comprise green and red LED's, and if a green illuminating radiation is used as the illumination source 31, LED's 77 may comprise blue and red LED's.

The tri-colour LED's **77** are each capable of providing an illumination radiation in one of three colours, typically red, green or blue, as controlled via the tri-colour sequence generator. Alternatively, a plurality of LED's in three groups, each group providing illumination in one of the desired colours, may be provided. The LED's **77** are located at the periphery of the interface **78** such that the LED's do not interfere with the other optical operations of the device **100.** In particular such operations include the transmission of the illuminating radiation for the confocal focusing operations, and also the transmission of reflected light from the object **26** to the main optics **41** to provide the 3D entity or the 2D colour entity. The LED's are mounted substantially orthogonally with respect to the interface **78**, and thus, as illustrated in Fig. 5C, light from each of the LED's 77 is transmitted by internal reflection with respect to the walls of the probe **90**, to the user interface end **79** of the probe.

Preferably, the device **100** according to a variation of the first embodiment is further adapted for providing improved precision of the colour data obtained therewith, in a similar manner to that described herein for the fourth embodiment, *mutatis mutandis.*

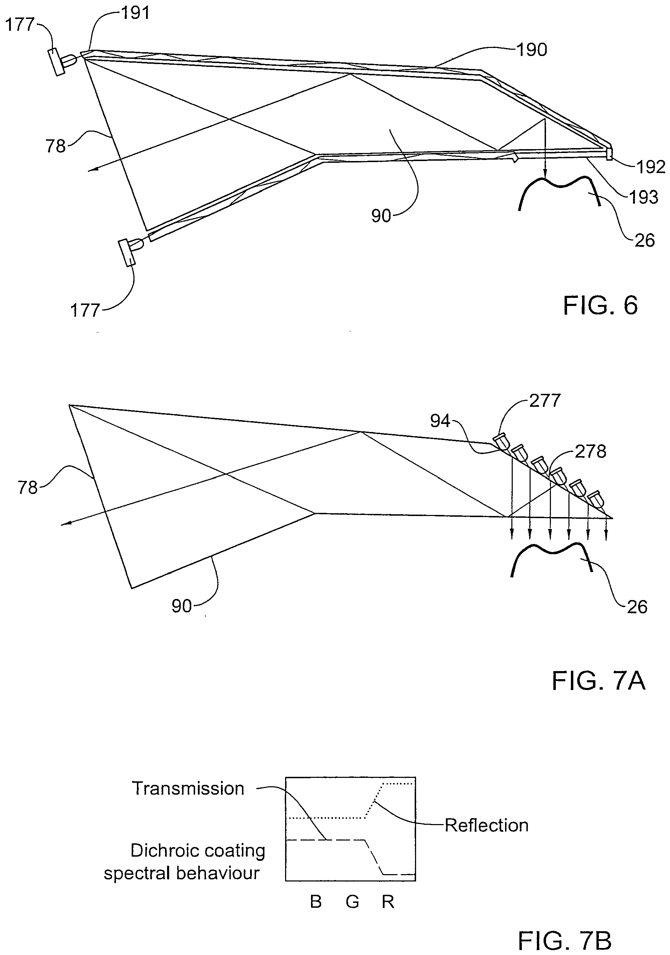

According to a second embodiment of the device **100**, the endoscope **46**, is also in the form of a probing member **90**, substantially as described with respect to the first embodiment, but with the difference that there are no LED's directly mounted thereon at the interface **78**, *mutatis mutandis.* In the second embodiment the delivery optics **73** is in the form of a disposable sleeve, shroud or sheath 190 that covers the outer surface the probing member **90**, as illustrated in Fig. 6. The sheath **190** is made from a waveguiding material, such as an acrylic polymer for example, capable of transmitting an illuminating radiation from the upstream face **191** of the sheath **190** therethrough and to the downstream face **192** thereto. The upstream face **191** is in the form of a peripheral surface around the interface **78.** The downstream face **192** is formed as a peripheral projection surrounding a window **193** comprised in said sheath **190.** The window **193** is in registry with the user interface end **79** of the probe **90.** A plurality of tri-colour LED's **177** for providing the coloured illumination to the object **26** are mounted on the device **100** just upstream of the sheath **190.** The tri-colour LED's **177** are each capable of providing an illumination radiation in one of three colours, typically red, green or blue, as controlled via the tri-colour sequence generator **74.** Alternatively, a plurality of LED's in three groups, each group providing one coloured illumination, may be provided. The LED's **177** are located outside of the main optics of the device **100**, and thus the LED's do not interfere with the other optical operations of the device **100** in particular including the transmission of the illuminating radiation for the confocal focusing operations, or in the transmission of reflected light from the object **26** to provide the 3D entity or the 2D colour entity. The LED's are mounted substantially opposite to the upstream face **191**, and thus, as illustrated in Fig. 6, light from each of the LED's **177** is transmitted by the waveguiding sheath **190** to downstream face **192** and thence to the object **26.** In this embodiment, the sheath **190** is particularly useful in maintaining hygienic conditions between one patient and the next, and avoids the need for sterilizing the probe **90**, since the sheath may be discarded after being used with one patient, and replaced with another sterilised sheath before conducting an intra-oral cavity survey with the next patient.

Preferably, the device **100** according to a variation of the second embodiment is further adapted for providing improved precision of the colour data obtained therewith, in a similar manner to that described herein for the fourth embodiment, *mutatis mutandis.*

In either one of the first or second embodiments, or variations thereof, a red laser may be used as the illumination source **28** for the main optics when creating the 3D entity. As such, this illumination means may also be used to obtain the red monochromatic image for the creation of the 2D colour image, by illuminating the object **26** and recording the image with the optical detector **60.** Accordingly, rather than tri-colour LED's or LED's or three different colours, it is only necessary to provide LED's adapted to provide only the remaining two colours, green and blue. A similar situation arises if the illumination source for the main optics **41** is a green or blue laser, wherein illuminating radiations in only the remaining two colours need to be provided, *mutatis mutandis.*

In these embodiments, the positioning of the illumination sources at the upstream end of the probe **90** where there is ample room rather than at the patient interface end **79** where space is tight.

According to a third embodiment of the device **100**, the endoscope **46** is also in the form of a probing member **90**, substantially as described with respect to the second embodiment with the following differences, *mutatis mutandis.* As illustrated in Fig. 7A, in the third embodiment the delivery optics **73** comprises a plurality of LED's **277** for providing the coloured illumination to the object **26.** In this embodiment, a red laser is used as the illumination source for the main optics when creating the 3D entity. As such, this illumination means is also used to obtain the red monochromatic image for the creation of the 2D colour image. Thus, the LED's **277** are each capable of providing an illumination radiation in either green or blue, as controlled via the tri-colour sequence generator **74.** The LED's **277** are located on the outer side of slanted face **94**, and thus the LED's do not interfere with the other optical operations of the device **100** in particular including the transmission of the illuminating radiation for the confocal focusing operations, or in the transmission of reflected light from the object **26** to provide the 3D entity or the 2D colour entity. The slanted face **94** comprises a dichroic coating **278** on the outer side thereof, which has relatively high reflectivity and low transmission properties with respect to red light, while having substantially high transmission characteristics for blue light and green light, as illustrated in Fig. 7B. Thus, as illustrated in Fig. 7A, light from each of the blue or green LED's **277** is transmitted, in turn, through the dichroic coating to interface **79** and thence to the object **26**, as controlled by the generator **74.** At the same time the dichroic coating permits internal reflection of the red radiation from the main optics **41** to the interface **79** and object **26**, and thus allows the 3D scan to be completed, as well as allowing the red monochromatic image of the object **26** to be taken by the device **100.** Optionally, rather than employing blue and green LED's, tricolour LED's may be used, and properly synchronized to illuminate with either green or blue light as controlled by generator **74.** Alternatively, the illumination source for the main optics **41** may be a green or blue laser, in which case the LED's are each capable of providing illumination in the remaining two colours, and in such cases the dichroic coating is adapted for allowing transmission of these remaining two colours while providing substantially high reflection for the illuminating laser of the main optics, in a similar manner to that described above for the red laser, *mutatis mutandis.*

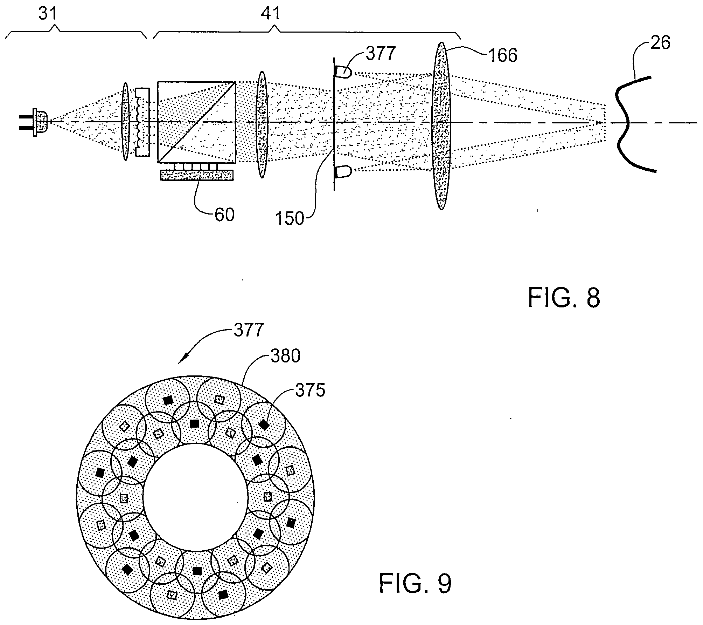

In a fourth embodiment of the device **100**, and referring to Fig. 8, tri-colour illumination is provided within the main focal optics **42**, in particular at the confocal system aperture stop, and facing the objective lens of the system. An advantage provided by this form of illumination is that the tri-colour illumination illuminates the object **26** through the downstream objective lens **142** in nearly collimated light, and thus the object illumination is highly uniform. The tri-colour light sources **377** may be mounted statically on the physical aperture stop at the aperture stop plane **150**, or alternatively they may be mounted on a retracting aperture stop, which also serves to stop down the system aperture in preview mode. In this embodiment, by placing the tri-colour light sources **377** at the aperture stop plane, wherein the light beam from the illumination source **31** narrows to a minimum within the main optics **41**, the external dimensions of the device **100** may still remain relatively compact.

Referring to Fig. 9, the tri-colour light sources **377** may comprise, for example, a plurality of tri-colour LED's **385** mounted onto a bracket **380.** The bracket **380** is typically annular, having a central aperture to allow illumination light from the illuminating unit **31** to pass therethrough and to the object **26**, and to allow light coming from the object **26** to pass therethrough and to the detection optics **60**, without being affected by the bracket **380.** At the same time, the bracket **380** positions the LED's in the required location upstream of objective lens **166.** The LED's are arranged in a spaced radial and circumferential manner as illustrated in Fig. 9 to provide the most uniform illumination of the object **26** possible with this arrangement. Typically, a red laser is used as the illumination source **31** for the main optics **41** when creating the 3D entity. As such, and as in other embodiments, this illumination means is also used to obtain the red monochromatic image for the creation of the 2D colour image. Thus, the LED's **385** are each capable of providing an illumination radiation in either green or blue, as controlled via the tri-colour sequence generator **74.** Alternatively, the illumination source for the main optics **41** may be a green or blue laser, in which case the LED's **385** are each capable of providing illumination in the remaining two colours, in a similar manner to that described above for the red laser, *mutatis mutandis.* Optionally, rather than employing blue and green LED's, tricolour LED's may be used, and properly synchronized to illuminate with either green or blue light as controlled by generator **74.** Further optionally, the LED's **385** may be used to provide, sequentially, all the required coloured illuminations, typically red, green and blue. Alternatively, the LED's **385** each provide illumination in one of at least three colours. Thus, some of the LED's **385** provide a blue illumination, while other LED's **385** provide green illumination, while yet other LED's **385** provide red illumination.

Preferably, the device **100** according to a variation of the fourth embodiment is further adapted for providing improved precision of the colour data obtained therewith. In this connection, the device **100** according to this variation of the fourth embodiment is adapted such that the tri-colour light sources **377** each illuminate the object **26** with as wide a depth of field as possible, i.e., at a low numerical aperture. Thus, each set of light sources **377** of the same colour, for example blue, illuminates a particular depth of the object **26** in the z-direction while substantially in focus. In contrast, the numerical aperture of the confocal system itself is relatively high to maximize accuracy of the depth measurements, and thus provides a relatively narrower depth of field.

Advantageously, the optical system downstream of the light sources **377**, in this embodiment the objective lens **166**, is chromatic, and in particular maximizes the chromatic dispersion therethrough. Alternatively or additionally, a chromatic dispersion element, for example an optically refractive block of suitable refractive index, may be provided along the optical path between the light sources **377** and the object **26.** Thus, each one of the different-coloured light sources **377** illuminates a different portion of the object **26** along the z-direction. The light sources **377** providing the blue illumination illuminate in focus a portion of the object **26** closest to the device **100**, and the light sources **377** providing the red illumination illuminate in focus a portion of the object **26** furthest from the device **100.** At the same time, the light sources **377** providing the green illumination illuminate in focus a portion of the object **26** intermediate the blue and red portions, and a non-illuminated gap may exists between the red and green, and between the green and blue illuminated portions, the depth of these gaps depending on the dispersion characteristics of the downstream optics. Advantageously, the light sources **377** are also adapted for providing illumination in colours intermediate in wavelengths such as to illuminate the aforesaid gaps in focus. Thus, the LED's **385** may be adapted for providing both such additional coloured illumination, or some of the LED's **385** may be adapted to provide coloured illumination at a first intermediate wavelength, while another set of LED's **385** may be adapted to provide coloured illumination at a second intermediate wavelength. For example, the first intermediate wavelength provides an illumination in aqua, and thus illuminates in focus at least a part of the gaps between the blue and green illuminated focused zones of the object **26**, while the second intermediate wavelength provides an illumination in amber, and thus illuminates in focus at least a part the gaps between the green and red illuminated focused zones. Of course, additional light sources may be used to provide further intermediate wavelengths and thus provide further depth cover illumination, in focus, of the object.

While the device **100** is used as a viewfinder, typically prior to taking a depth and colour scan of the object **26**, the above arrangement using at least five different coloured illuminations at a low numerical aperture, enables a much clearer and focused real-time colour image of the object **26** to be obtained. Thus when in operation in viewfinder mode (also known as "aiming mode", prior to the 3D scan event, while the dental practitioner is in the process of aiming the scanner onto the target dental surface, for example) the device **100** according to this variation of the fourth embodiment repeatedly illuminates the object **26** in cycles, wherein in each cycle the object **26** is separately illuminated in each of the five colours blue, aqua, green, amber, red, in quick succession, and each time a monochromatic image is obtained by the monochromatic image sensor in **60.** Each set of five monochromatic images is then analysed to provide a composite colour image, and this image is then displayed in substantially real time in the viewfinder display window in the control software, so that the succession of such composite images gives the appearance of a substantially real-time colour video feed of the object **26.**

Each of the monochrome images in any particular set corresponds to a particular illumination colour or wavelength, and thus the zone(s) of the object **26** within the depth of field corresponding to this illumination will be in focus, while the other parts of the object **26** will appear out of focus. Thus, each such image in the aforesaid set of images will contain a portion which has high precision focused image of a part of the object, for the particular illumination wavelength.

In forming a composite image for each set of images, the images are combined in such a way as to maximize the precision of the focused image and corresponding colour thereof. Thus, for example, suitable algorithms may be applied to each of the five images of a set to distinguish between the focused and unfocused the areas thereof. Such algorithms may employ, for example, techniques which apply FFT techniques to areas of the images, and which search for high frequency portions which correspond to focused areas. In any case, such algorithms, as well as software and hardware to accomplish the same are well known in the art. Then, the focused areas of each of the five images are merged to provide a monochrome composite substantially focused image of the object. Next, the images obtained using the red, green and blue illuminations are combined and converted to a corresponding luminescence/chroma (Y/C) image, and techniques for doing so are well known in the art. Finally, the luminescence component of the luminescence/chroma (Y/C) image is replaced with the aforesaid corresponding composite focus image, and the resulting new luminescence/chroma image is then transmitted to the display in the viewfinder.

For each set of images, prior to combining the corresponding red, green and blue images, these are preferably first scaled to compensate for magnification effects of the different wavelengths. Thus, the green image, and more so the blue image, needs to be scaled up to match the red image.

When the user is ready to take a depth and colour scan of the object **26**, having steered the device **100** into position with the aid of the viewfinder, the device **100** takes a depth scan in the z-direction as described herein, and either before or after the same, but in quick succession one with the other, takes a colour scan in a similar manner to that described above for the viewfinder mode, *mutatis mutandis.* Subsequently, the colour data and the depth data of the two scans can be combined to provide the full spatial and colour data for the surface scanned.

Advantageously, one or more colour scans may also be taken during the depth scan, and/or at the beginning and at the end of the depth scan. In one mode of operation, the depth scan is obtained by displacing the objective lends **166** along the z-direction in a continuous or stepped motion. Multiple colour scans can then be obtained by associating the colour sources **377** with the objective lens, so that these are also displaced along the z-direction. Accordingly, as the light sources **377** are moved in the z-direction towards the object **26** during the depth scan, at each different z-position in which a set of images is taken (concurrently with or alternately with the depth scan), each one of the coloured illuminations - red, green, blue and intermediate wavelengths - illuminates a progressively deeper part of the object along the z-direction. Of course, in some cases it is possible that at the downstream end of the depth scan the green and red illuminations completely overshoot the object **26**, and the corresponding images may be discarded or otherwise manipulated to provide a composite colour image at this station. Thus, a plurality of colour images can be obtained, each based on a different z-position, so that each illumination wavelength is used to illuminate in focus a different part (depth) of the object **26.** Advantageously, suitable algorithms may be used to form a composite colour image of the set of colour images associated with a particular z-scan of the object **26** to provide even more precise and accurate colour image, than can then be combined with the depth data.

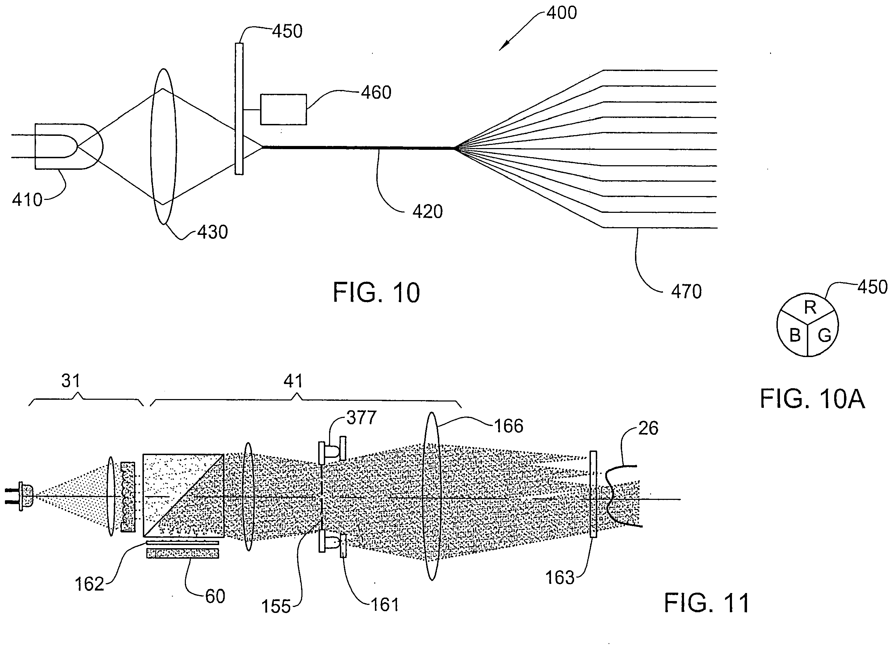

Alternatively, and referring to Fig. 10, the tri-colour light sources **377** may be replaced with a rotating filter illumination system **400.** The system **400** comprises a while light source **410**, such as for example white phosphorus InGaN LED's, and the light therefrom is focused onto an optical fiber bundle **420** by means of condenser optics **430.** Between the condenser optics **430** and the fiber bundle **420** is provided a rotating tri-colour filter **450.** As best seen in Fig. 10A, the filter **450** is divided into three coloured sections, comprising blue, green and red filters on adjacent sectors therein. The fiber bundle **420** is flared at the downstream end **470** to form the desired illumination pattern. Optionally, the downstream end **470** of the fibers may be mounted onto an annular bracket similar to bracket **380** illustrated in Fig. 9, at the aperture stop plane of the confocal optics. A suitable motor **460**, typically a stepper motor for example, drives the rotating filter such as to sequentially present each coloured filter to the light passing from the condenser optics **430** to the fiber bundle **420**, as synchronized with the sequence generator **74** (Fig. 1) to enable the detection optics **60** to capture images of the object 26 when selectively illuminated with each of the three colours. Optionally, if a red, blue or green illuminating radiation is used as the illumination source **31** for the main optics **41** when creating the 3D entity, then the rotating filter **450** only requires to comprise the remaining two colours, as discussed above for similar situations regarding the LED's, *mutatis mutandis.*

Preferably, the device **100** according to this variation of the fourth embodiment may be further adapted for providing improved precision of the colour data obtained therewith, in a similar manner to that described herein for another variation of fourth embodiment, *mutatis mutandis.* In particular, the filter 450 is divided into five (or more if desired) coloured sections, comprising blue, aqua, green, amber and red filters on adjacent sectors therein.

A fifth embodiment of system **100** is substantially similar to the fourth embodiment as described herein, with the following difference, *mutatis mutandis.* In the fifth embodiment, and referring to Fig. 11, polarizer are provided at two locations in order to increase the image contrast. A first polarizing element **161** is located just downstream of the light sources **377** so as to polarize the light emitted from the light sources **377.** A second polarizing element **162** is located just upstream of the image sensor of the detection optics **60**, and is crossed with respect to the first polarizing element **161.** Further, a quarter waveplate **163** is provided just upstream of the object **26**, i.e. at the downstream end of the endoscope **46** (Fig. 4A). The first polarizing element **161** is typically annular, having a central aperture to allow illumination light from the illuminating unit **31** to pass therethrough and to the object, and to allow light coming from the object **26** to pass therethrough and to the detection optics **60**, without being affected by the polarizing element **161.** However, light that is reflected from the object **26** returns to the main confocal optics **42** in a crossed polarization state due to the effect of the quarter waveplate **163**, and thus reaches the detection optics **60** at substantially full intensity. However, any light reflected from the objective lens **166** of the confocal optics **42** is reflected at the same polarization state, and is therefore filtered out by the crossed polarizing element **162.** This arrangement serves as an effective signal to ghost enhancement system.

Preferably, the device **100** according to a variation of the fifth embodiment is further adapted for providing improved precision of the colour data obtained therewith, in a similar manner to that described herein for the fourth embodiment, *mutatis mutandis.*

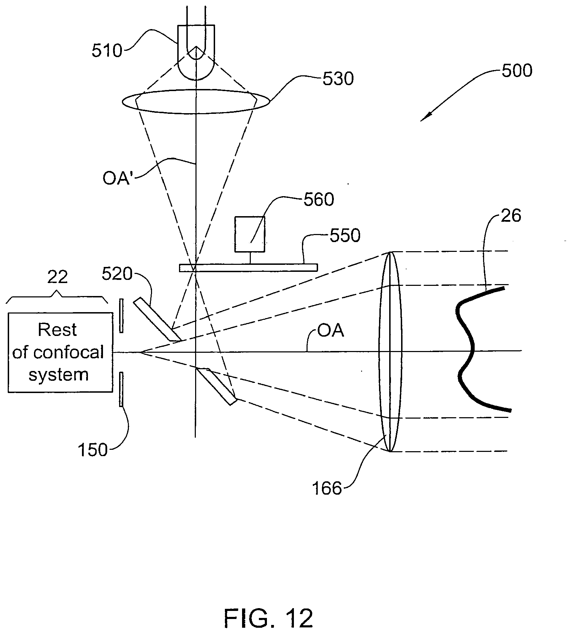

A sixth embodiment of the system **100** is substantially as described for the fourth embodiment, with the following difference, *mutatis mutandis.* In the sixth embodiment, and referring to Fig. 12, the tri-colour light sources **377** are replaced with a rotating filter illumination system **500.** The system **500** comprises a while light source **510**, such as for example white phosphorus InGaN LED's, and the light therefrom is focused onto a mirror **520** by means of condenser optics **530.** Between the condenser optics **530** and the mirror **520** is provided a rotating tri-colour filter **550**, which is similar to the filter **450** illustrated in Fig. 11, and thus comprises three coloured sections, comprising blue, green and red filters on adjacent sectors therein, and is actuated by motor **560.** The optical axis **OA of** the confocal optics **41** is orthogonal to the optical axis **OA'** of the light source **510** and condenser optics **530.** The mirror **520** is mounted between the aperture stop plane and the objective lens **166** of the confocal optics, and at an angle to the optical axis **OA** thereof and to the optical axis **OA'** of the light source **510** and condenser optics **530.** The mirror **520** is typically annular, having a central aperture aligned with optical axis **OA** to allow illumination light from the illuminating unit **31** to pass therethrough and to the object **26**, and to allow light coming from the object **26** to pass therethrough and to the detection optics **60**, without being affected by the mirror **520.** At the same time, the mirror **520** has sufficient reflecting surface to reflect light from the source **510** via objective lens **166** and to the object **26.** Optionally, if a red, blue or green illuminating radiation is used as the illumination source **31** for the main optics **41** when creating the 3D entity, then the rotating filter **550** only requires the remaining two colours, as discussed above for similar situations, *mutatis mutandis.*

Preferably, the device **100** according to a variation of the sixth embodiment is further adapted for providing improved precision of the colour data obtained therewith, in a similar manner to that described herein for the fourth embodiment, *mutatis mutandis.*

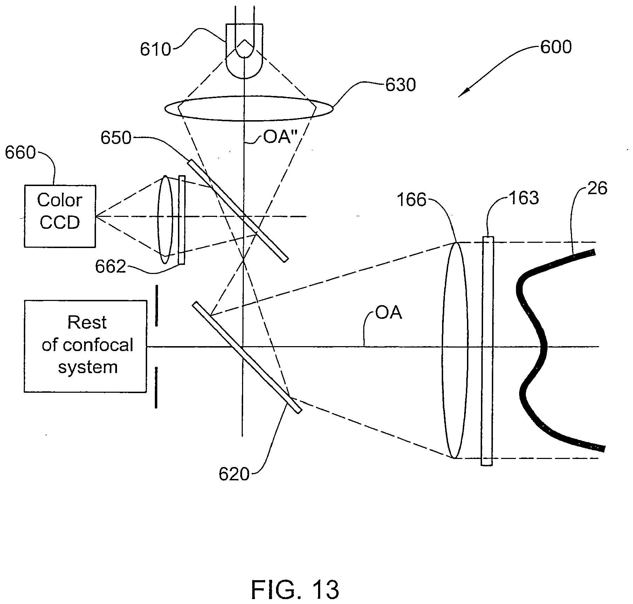

According to a second technique for providing the aforesaid 2D colour image, the object **26** is illuminated with a white light, and a colour CCD is used for receiving the light reflected from the object **26.** Thus, a seventh embodiment of the system **100** comprises a white light illumination system **600**, illustrated in Fig. 13. The system **600** comprises a while light source **610**, such as for example white phosphorus InGaN LED's, and the light therefrom is directed onto a flip mirror **620** via a polarizing beam splitter **650** by means of condenser optics **630.** The optical axis **OA** of the confocal optics **41** is orthogonal to the optical axis **OA"** of the light source **610** and condenser optics **630.** The mirror **620** is mounted between the aperture stop plane **155** and the objective lens **166** of the confocal optics, and at an angle to the optical axis **OA** thereof and to the optical axis **OA"** of the light source **610** and condenser optics **630.**

The mirror **620** is adapted to flip away from optical axis **OA** when the device **100** is being used for obtaining the 3D entity E. This allows illumination light from the illuminating unit **31** to pass therethrough and to the object **26**, and to allow light coming from the object **26** to pass therethrough and to the detection optics **60**, without being affected by the mirror **620.** When it is desired to take a 2D colour image, the mirror **620** is flipped down to the position shown in Fig. 13. Polarizing beam splitter **650** that polarizes white light from the source **610** and allows the same to pass therethrough and to mirror **620**, and thence to the object **26** via the confocal objective **166** and broadband quarter wave plate **163.** Light that is reflected from the object **26** returns to the mirror **620** in a crossed polarization state due to the effect of the quarter waveplate **163**, and thus reaches the colour CCD **660** (and associated detection optics - not shown) at substantially full intensity. However, any light reflected from the objective lens **166** of the confocal optics **42** is reflected at the same polarization state, and is therefore filtered out by a crossed polarizing element **662** just upstream of the CCD **660.** This arrangement serves as an effective signal to ghost enhancement system.

Alternatively, the CCD of the detection optics **60** is a colour CCD and is also used for the 2D scan. In such a case, flipping mirror **620** is replaced with a fixed mirror having a central aperture similar to mirror **520**, having a central aperture, as described for the sixth embodiment, mutatis mutandis.

In the seventh embodiment, the image capture device **80** and processing software **82** (Fig. 4b) automatically provide a 2D colour image comprising an array of data points having location **(X,Y)** and colour **(C)** information for each pixel of the image.

According to a third technique for providing the 2D colour image, the object is illuminated with a white light, and the light reflected from the object **26** is passed sequentially through one of three different coloured filters such as red, green ad blue. Each time a monochromatic image corresponding to each colour is captured via CCD **68** and the image capture device **80** (see Figs. 4A, 4B). Suitable processing software **82** combines the three images to provide a 2D colour image comprising an array of data points having location **(X,Y)** and colour **(C)** information for each pixel of the image.

According to a fourth technique for providing the colour image, the main illumination source **31** of device **100** comprises suitable means for providing the three different coloured illuminations. In one embodiment, the illumination source **31** comprises three different lasers, each one providing an illumination radiation at a different desired colour, red green or blue. In another embodiment, a suitable white light illumination means is provided, coupled to a suitable rotating tri-colour filter, similar to the filters described above, *mutatis mutandis.* In each case, suitable control means are provided, adapted to illuminate the object **26** with each coloured radiation in turn, and the 2D coloured image is obtained in a similar fashion to that described above, *mutatis mutandis.* The object is also illuminated with one of the coloured illuminations in order to provide the 3D surface topology data.

In each of the embodiments described herein, the illumination radiation that is used for obtaining the 2D colour image is injected into the optical axis OA of the confocal optics **42** without affecting the operation thereof or degrading the 3D image capture.

The endoscope **46**, the illumination unit **31**, the main optics **41**, colour illumination **71** and tri-colour sequence genetrator are preferably included together in a unitary device, typically a hand-held device. The device preferably includes also the detector optics **60**, though the latter may be connected to the remainder of the device via a suitable optical link such as a fiber optics cable.

For all embodiments, the data representative of the surface topology and colour, i.e., entity **I**, may be transmitted through an appropriate data port, e.g. a modem **88** (Fig. 4B), through any communication network, e.g. telephone line **90**, to a recipient (not shown) e.g. to an off-site CAD/CAM apparatus (not shown).

By capturing, in this manner, an image from two or more angular locations around the structure, e.g. in the case of a teeth segment from the buccal direction, from the lingual direction and optionally from above the teeth, an accurate colour three-dimensional representation of the teeth segment may be reconstructed. This may allow a virtual reconstruction of the three-dimensional structure in a computerized environment or a physical reconstruction in a CAD/CAM apparatus.

While the present device has been described in the context of a particular embodiment of an optical scanner that uses confocal focusing techniques for obtaining the 3D entity, the device may comprise any other confocal focusing arrangement, for example as described in WO 00/08415. In fact, any suitable means for providing 3D scanning can be used so long as the 3D scan and the colour 2D scan correspond substantially to the same object or portion thereof being scanned, and the same frames of references are maintained. Typically the scans are executed in relatively quick succession, and by the same or different image capturing means such as CCD's that are arranged such that the colour 2D image substantially corresponds to the 3D entity. This enables colour values at particular x, y coordinates of the 2D colour image to be matched to the same x, y coordinates of the 3D image which also have a z coordinate.

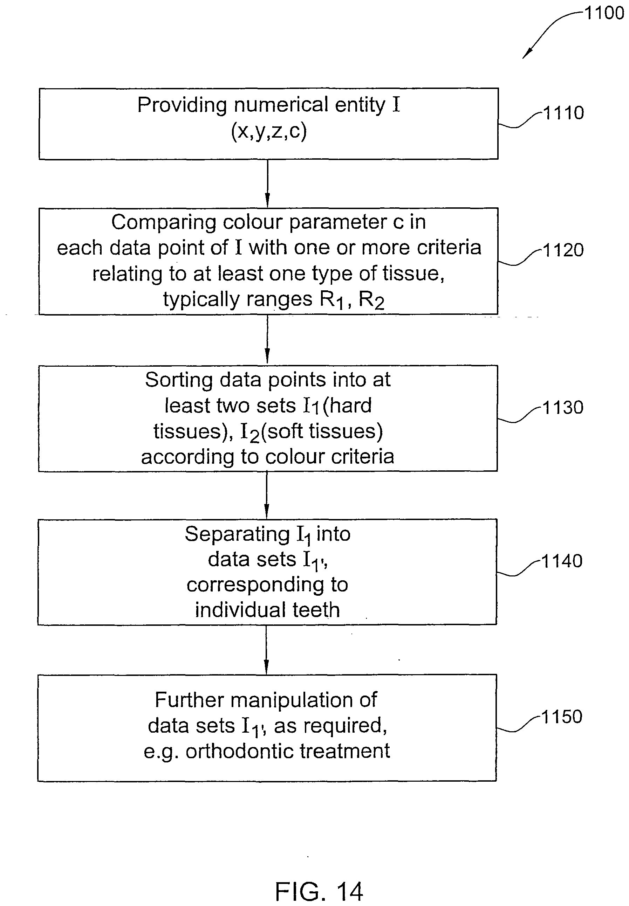

Referring to Fig. 14, the method, generally designated **1100,** is particularly adapted for automatically differentiating one tissue from another tissue within the oral cavity. The first step **1110** is to obtain numerical entity **I** comprising a data set representing the surface geometry and colour of at least the portion of the intraoral cavity that contains the area of interest, herein referred to as the target surface. The target surface typically comprises both hard tissues, such as the teeth, and soft tissues which include at least one of the gums, tongue, cheeks and lips. Each data point in the numerical entity **I** comprises surface coordinates of the tissue, typically expressed as Cartesian **(x, y, z)** coordinates, plus a colour parameter relating to the colour c of the tissue at these surface coordinates. The colour value for **c** can be conveniently expressed in component form in terms of the red, green and blue components (RGB), and in particular in terms of a colour component coefficient. For example, a red coefficient ratio Rc, may be defined as the intensity, of the red component divided by the average intensity of the red, green and blue components, and is useful in differentiating between the hard and soft tissues. The entity **I** may be obtained, for example, using the device described herein with respect to Figs. 1 to 13.

In the next step **1120**, the value of the colour parameter c is analysed for each data point in **I**, and compared with at least one colour criterion, and typically with at least two colour ranges **R₁**, **R₂**. The ranges **R₁**, **R₂** each represent the values of the colour parameter expected for one or another of the two tissues, such as the teeth and gums. For example, the colour range **R₁** for the teeth will include values for **c** typically associated with the hard tissues including the teeth, comprising all appropriate shades appropriate for enamel, dentine, pulp and other parts of teeth. Similarly, the colour range **R₂** for the soft tissues will include values for **c** typically associated with gums, cheeks, lips and the tongue including all appropriate shades of pink and red associated with these tissues, including when the tissues are at least partially drained of blood, as may happen, for example when the tissues are anaesthetised. In some cases it may be appropriate to compare the value of the colour parameter **c** with a specific value, for example a single (R, G, B) or Rc value, rather than a range of values.

Table I lists typical RGB values measured for gums, lips and teeth of a particular patient. As may be seen from Table I, values of **Rc** significantly greater than unity indicate that the red component is dominant, which is the case for soft tissues in general. Hard tissues, on the other hand, have a more even distribution of colour components, resulting in an **Rc** value very close to unity.

**Table I**

| **Intraoral RGB Values measured for a Patient** | | | | |
|---|---|---|---|---|
| **Tissue** | **Red component** | **Green component** | **Blue component** | **Rc=3^{∗}R/(R+G+B)** |
| **Gum 1** | 76 | 28 | 28 | 1.6 |
| **Gum 2** | 119 | 79 | 80 | 1.28 |
| **Lower lip** | 165 | 120 | 130 | 1.2 |
| **Premolar** | 168 | 175 | 167 | 0.99 |
| **Incisor** | 172 | 174 | 170 | 1.0 |

Thus, an exemplary range for **R₁** may be from about 0.9 to about 1.1, while an exemplary range for **R₂** may be from less than about 1.2 to a maximum of 3.0.

The ranges **R₁**, **R₂** should preferably be sufficiently spaced one from the other and not overlap to facilitate distinction between the different tissues and to prevent ambiguity. At the same time, each range should include all possible variations of colour that may be expected for each of the tissues involved.

The actual values of the ranges **R₁**, **R₂** may vary between individuals. For example, some individuals may have yellowing teeth and pale soft tissues, while others may have milky white teeth and reddish complexion. Optionally, it is possible to pre-calibrate the ranges **R₁**, **R₂**, for example by scanning an area that is purely soft tissue, and another dental area that is purely hard tissue, and using the colour values for the two scans as datum values for the two ranges **R₁**, **R₂**.

Optionally, each tissue type may also be associated with more than one range of colour values, and thus each one of **R₁**, **R₂** may comprise a set of ranges. For example, **R₂** may actually include four separate ranges, each relating to the colour variations of one of the gums, cheeks, lips and tongue. Also, **R₁** may include a number of separate ranges, one range representative of the variations in the colour of natural teeth, while the other ranges relate to colours associated with prostheses and/or with fillings, particularly when made from materials which do not give a natural appearance to the prostheses, for example gold crowns or amalgam fillings.

In the next step **1130,** the data points for entity I are sorted into at least two sub-data sets, **I₁**, **I₂**, according to the colour criteria, that is, for example, whether the value of the colour parameter of each data point is within **R₁** or **R₂**, respectively. Thus, **I₁** will contain all data points in which the colour parameter thereof corresponds to the colour of teeth, and thus should comprise the coordinates relating to the surfaces of teeth only within the original entity **I** (optionally including prostheses and fillings). Similarly, **I₂** should contain all data points in which the colour parameter thereof corresponds to the colour of the soft tissues, and thus should comprise the coordinates relating to the soft tissues only within the original entity **I.**

In a modification, it may only be necessary or desired to identify one tissue, such as the teeth for example, and disregard all data not relating to this tissue. In such cases, it is only necessary to compare the value of the colour component of each data point to a single colour criterion, such as for example a predetermined range **R₁** relating to the teeth only, and then separate out these data points from the entity **I** to provide an entity **I₁** that comprises data relating only to the teeth. Of course, it may also be desired to include in this data set artificial teeth and also fillings that do not have a natural colour, and thus the range **R₁** may optionally include the appropriate values for the colour parameter relating thereto.

Once the original entity **I** has been separated into two entities, or wherein an entity **I₁** has been created from the original entity **I** comprising only the tissue of interest, the new entity may be further manipulated as desired. In step **1140,** for example, when the new entity **I₁** comprises only teeth-related data, each individual tooth may be identified therein. In such a situation, the entity **I₁** is further separated out into a plurality of smaller entities **I₁,** each of which relates to a separate tooth. Typically, the separation of **I₁** into **I**_{**1**'} is automatically effected using any suitable algorithm

In step **1150,** after the data relating to the individual teeth has been properly sorted, further manipulation may be carried out for each of the individual data sets of the entities **I₁**, for example to simulate a particular orthodontic treatment for the teeth.

This method may also be applied to the identification of a finish line profile for a crown or bridge prosthesis.

The finish line may be regarded as the circumferential junction or shoulder between the upper prepared portion of the tooth and the lower unprepared portion of the tooth. The finish line may be above or below the visible gum line, i.e. the exterior visible line of gingival tissue which circumferentially surrounds the tooth. Frequently, the finish line is below the visible gum line and is uneven, i.e. the finish line varies in height along the circumferential direction and can rise or fall on the order of several millimetres in the generally vertical direction. The finish line may even, in some cases, extend as far downwardly as the attachment line, i.e. the circumferential line defined by the neck of the tooth and its immediately adjacent gingival tissue below the aforementioned visible gum line. As with the finish line, the attachment line is uneven and also typically varies several millimetres in height along the circumferential direction. The contour of the attachment line varies from tooth to tooth, as well as from patient to patient, and is not readily visible or accessible to the dentist because it is below the visible gum line. In such cases, a retention ring or wire, made of an elastically deformable material, may be placed around the preparation to retract the gum tissue around the preparation. The ring thus in many cases exposes at least part of the emerging profile - the surface of the tooth between the finish line and the gum.

The ring thus adopts a profile which may often be substantially similar to that of the finish line. By having the ring coloured sufficiently differently to the colour of the teeth or soft tissues, say in blue, it is relatively straightforward to separate out from the entity **I** all data points having a colour component with a value in a specific range corresponding to the colour of the ring. Identification of the ring itself provides a useful starting point for suitable algorithms that are then applied to determine the location and geometry of the finish line. Such algorithms are known and typically attempt to identify features commonly found with finish lines such as for example, a discontinuity in the slope of the tooth surface, or a mound-shaped projection corresponding to the preparation. Moreover, separation of the hard tissue from the soft tissue results in a smaller data base that needs to be analysed to identify the finish line. In particular, when the data set has been separated into entities **I₁**, then only the specific entity **I₁** corresponding to the ring needs to the analysed for the finish line, as this entity corresponds to the preparation.

In all variations of this method, the comparison of value for the colour parameter **c** with an appropriate range, and the sorting of data points into one or more data sets according to this comparison can be executed with any suitable computer with the aid of a suitably constructed program. The manipulation of the entities at each stage with respect to the computer may be manual, interactive, or partially or fully automated.

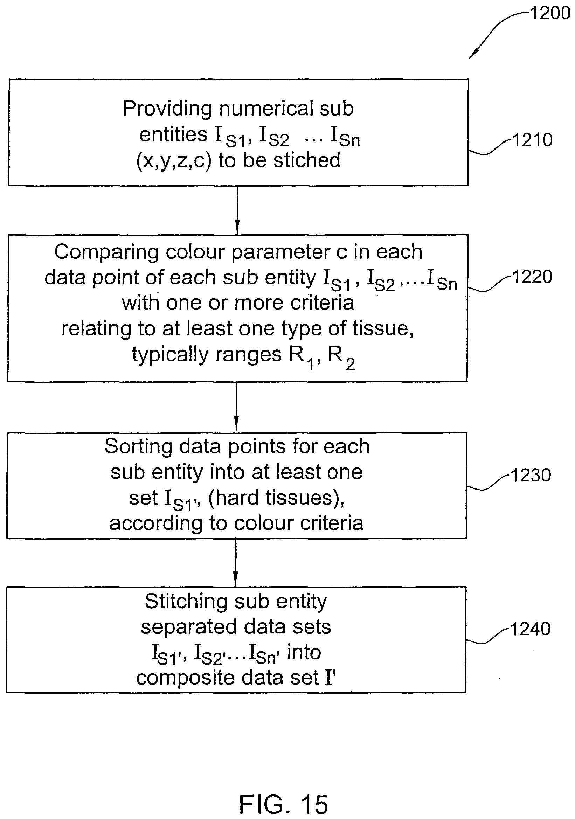

Referring to Fig. 15, the method, generally designated **1200,** finds particular use for accurately stitching data sets of overlapping areas scanned within the intra-oral cavity. In the first step **1210,** a suitable scanner, as described herein, for example, may be used for obtaining high resolution numerical sub-entities **(IS₁, IS₂,...ISₙ)** so representative of the surface topography and colour of zones **(A₁, A₂,...Aₙ)** within the intra-oral cavity. The zones that are scanned should together fully span the target zone of interest. Some of these zones may overlap with all the other zones, while other zones may only overlap with one or two other zones, and thus theoretically the corresponding entities should have a portion of their data identical to one another within the overlap zone. However, since the scanner itself moves in orientation and position from scan to scan, the coordinates relating to these overlap zones will usually vary between adjacent scans, even though they relate to the same spatial feature. Nevertheless, by identifying the overlap zone within the entities, the spatial relationship between the entities may be established, and the various entities stitched together or combined to form a global numerical entity that comprises the full geometry and colour representation of the target zone. The larger the overlap zone, the greater the accuracy with which the different zones may be synchronized together spatially.

In prior art methods, the overlap zones may be identified by numerically transforming the coordinates of an entity associated with one zone - by a series of translations and rotations - and in each case comparing the data set with the data set of another entity. This process is repeated until at least a portion of the data from the first entity coincides with at least a portion of the data from another entity. At this point, the data sets comprising the two sub-entities can be combined by adding both sets of coordinates, and discarding every data point that is repeated. However, some ambiguity may occur when using such a technique if a part of the intra-oral cavity (corresponding to part of the overlapping scanned data in some of the entities) moves in relation to other parts. For example, between one scan and another scan, part of the cheek may move relative to the teeth. It is then problematic to construct a composite entity comprising both scans since at least a part of the tissues (in this example the cheek) will be associated with two different data portions representative of the relative movement between scans.

With this method, a method for stitching different data sets for the intraoral cavity is provided, in which the actual stitching technique is applied to data points corresponding to the hard tissues therein. Accordingly, in a second step **1220** of the method, the hard tissues including the teeth, fillings and prostheses are differentiated from the soft tissues including the gums, cheeks, lips and tongue. Substantially the same method as described above in the context of Figure. 14 may be utilized to identify the data, in each of the sub-entities **(IS₁, IS₂,...ISₙ)** that is associated with the hard tissues, *mutatis mutandis.* The data in these entities not corresponding to the hard tissues may de discarded or simply noted and set aside for "future reference, thereby providing modified entities **(IS'₁, IS'₂,...IS'ₙ)** comprising the data of interest relating to the hard tissues, for example.

In the next step 1230 of the method, the modified entities **(IS'₁, IS'₂,...IS'ₙ)** are then manipulated in a similar manner in which the original entities **(I₁,I₂,...Iₙ)** are manipulated in the prior art, *mutatis mutandis,* to register and then stitch the various modified entities **(IS'₁, IS'₂,...IS'ₙ)** together to provide a composite entity **I'** that comprises the data points corresponding at least to the hard tissues.

As an optional step, the data referring to the soft tissues may then be added to the composite entity **I'** as follows. Referring to the soft tissue data corresponding to each scan as entities **(IS"₁, IS"₂,...IS"ₙ),** each one of these entities is first manipulated in precisely the same manner as the corresponding entity of the group of modified entities **(IS'₁, IS'₂,...IS'ₙ)** was manipulated in order to stitch the latter together into **I'**. After this, the coordinates of each pair of entities within the group **(IS"₁, IS"₂,...IS"ₙ)** are compared in turn. Each pair of entities within the group **(IS"₁, IS"₂,...IS"ₙ)** is checked to determine whether there exist some data points in one entity having two coordinates, say (x, y) identical to corresponding data points in the other entity, but in which the (z) coordinates are different. All such data points in either one or the other entity are then disregarded. In this manner, a composite entity I" can be constructed for the soft tissues, which can then be added to, if desired to the composite entity I' of the hard tissues previously created.

Typically, course stitching of the original sub entities **(IS₁, IS₂,...ISₙ)** is first carried out, and when the approximate relationships between the sub entities is known, a next step is performed, comprising fine stitching the corresponding separated hard-tissue sub entities **(IS'₁, IS'₂,...IS'ₙ).**

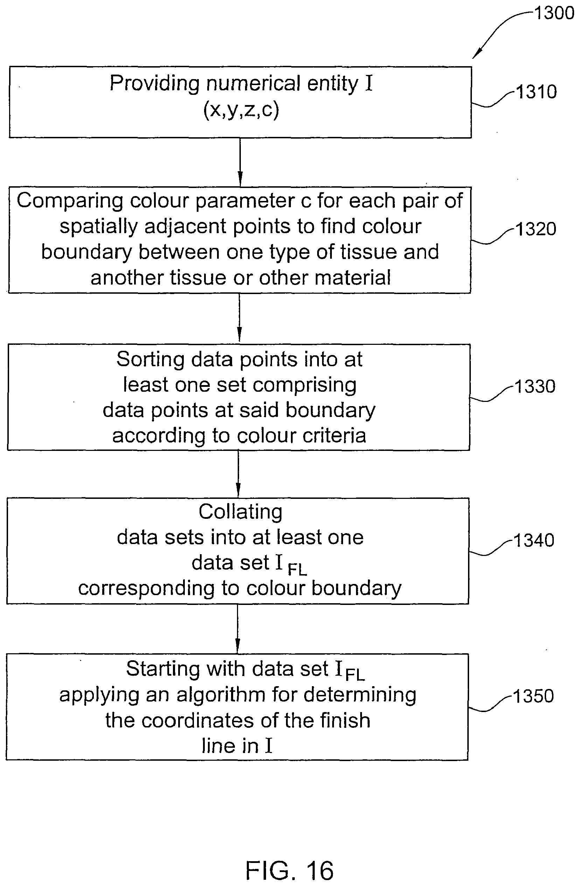

In the embodiment of the present invention, and referring to Fig. 16, the method, generally" designated **1300**, is used for automatically defining the finish line of a preparation. As described above, the finish line may be regarded as the circumferential junction or shoulder between the upper prepared portion of the tooth and the lower unprepared portion of the tooth. The finish line may be above or below the visible gum line, i.e. the exterior visible line of gingival tissue which circumferentially surrounds the tooth. In cases where the finish line protrudes from the gum, the complete circumferential extent of the finish line is visible and appears as a shade of white. This may be contrasted with the pink/red colour of the surrounding gums, and at the same time the finish line is assumed to be in close proximity to the gums. In such a case, the method of the invention is carried out as follows.

As with other methods, the first step **1310** is to provide a numerical entity **I** that describes the target area - in this case the part of the intraoral cavity that comprises the finish line - geometrically and with respect to colour. Preferably, the target area is confined to the tooth having the preparation, and possibly the adjacent teeth. Then, in step **1320**, an algorithm is applied to every pair of spatially adjacent data points, for example, wherein the value of the colour parameter c of each of these points are compared one with the other, or with respect to some colour criteria. When it is determined that the difference in colour values is greater than a predetermined threshold, it is then assumed that the pair of data points are on opposite sides of a boundary between two tissues of different colour, in particular between the edge of the tooth and the gum. Alternatively, the value of parameter c is compared with two predetermined ranges, **R₁, R₂,** wherein each range corresponds to a colour associated with one or the other of teeth and gums. Then, wherever there is a pair of adjacent data points in which one data point has a value for parameter c within **R₁** and the other data point has a value for parameter c within **R₂,** once again these two data points are considered to be on either side of the boundary between a tooth and the gum. The process is repeated for each adjacent pair of points in the entity **I**, thereby providing in step **1330** another numerical entity **I_{FL}** representative of the gum line and comprising topographic (as well as colour) information relating thereto.

Identification of the gum line itself provides a useful starting point for suitable algorithms that are then applied in step **1340** to determine the location and geometry of the finish line. Such algorithms typically attempt to identify features commonly found with finish lines, as has been described herein for the first embodiment. Thus, the algorithm is applied to the entity I, but staring with the surface data thereof corresponding to entity **I_{FL}.**

In cases where the finish line is partially or fully located below the gum, a suitable ring may be placed between the neck of the tooth preparation and the gum such as to retract the latter and expose the finish line. The method may then be applied with a modification in that the boundary between the tooth material and the ring material is searched for, in a similar manner to that described regarding the boundary between the tooth material and gum, *mutatis mutandis,* which provides a starting point for algorithms that are then applied to identify the finish line.

Preferably, where the entity **I** is viewable as a two dimensional colour image on a screen, the method optionally further comprises the step of displaying on such an image of the entity **I** the finish line entity **I_{FL},** preferably in a colour having high contrast with respect to **I,** to enable the practitioner to check the result.

The method according to this embodiment may be modified to separate the soft tissues from the hard tissues, once the demarcation line between the two tissues is known, as determined above.

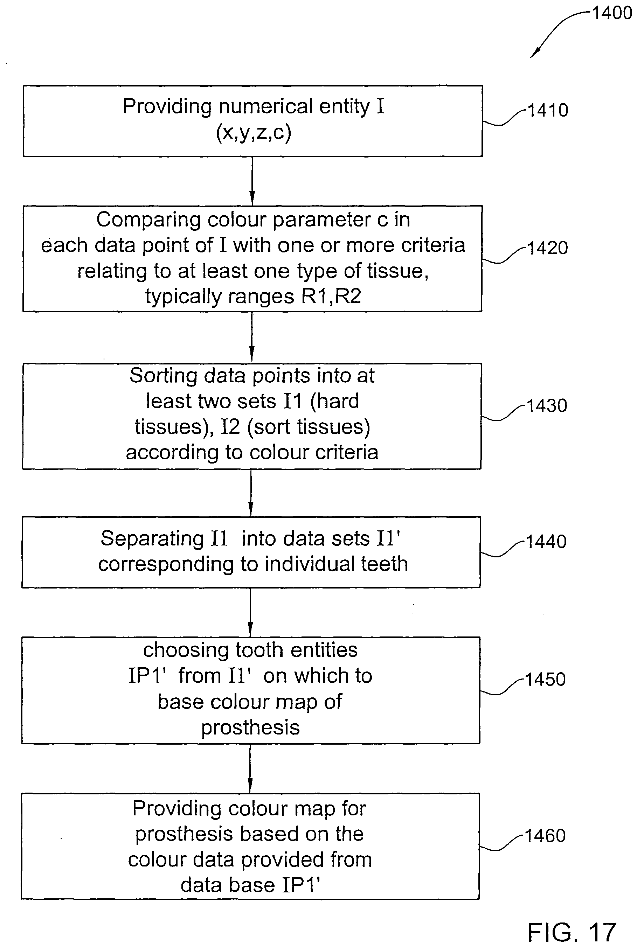

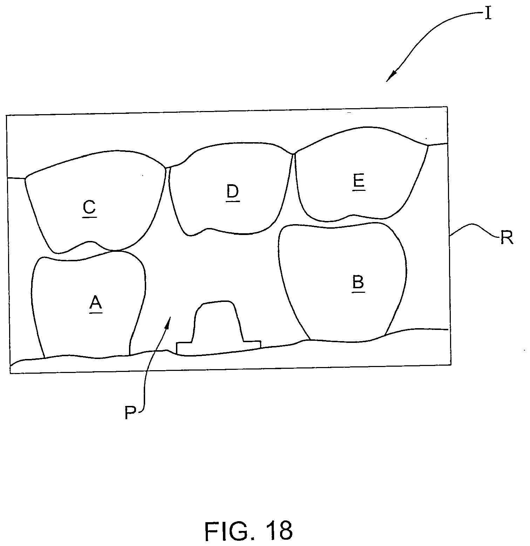

Referring to Fig. 17, the method, generally designated **1400,** is particularly applied to the shading of a prosthesis. As with the other methods, the first step **1410** involves the creation of a numerical entity **I** of the region of interest within the oral cavity. Referring to Fig. 18, in this case, the region of interest **R** includes the preparation area **P** of the intraoral cavity in which the prosthesis is to be mounted, including adjacent teeth thereto, **A, B** as well as the teeth **C, D, E** opposite thereto on the opposing jaw. The region of interest R may optionally or alternatively include at least one tooth or teeth on the other side of the same jaw corresponding to the tooth or teeth that the prosthesis is designed to replace. The desired data in this embodiment that it is desired to create from the entity **I** relates to shading information for the prosthesis such as to provide a natural-looking appearance thereto that is compatible with the other teeth of the patient. The shading characteristics of the prosthesis according to. the invention are to be based on the shading characteristics of at least one tooth of the patient, and thus share the same colour, variations in hue and other features such as reflective qualities and translucency, that may be found on one or more teeth of the patient, according to any desired set of rules or algorithm.

In the next steps **1420, 1430, 1440,** the entity **I** is manipulated such as to extract the data corresponding to the teeth only, and to separate this data into a set of discrete entities **I₁,** each of which represents an individual teeth, substantially as described regarding steps **1120, 1130 and 1140,** respectively, above, *mutatis mutandis.*

Then, in step **1450**, the decision is taken regarding which teeth are to be considered for modelling the shading of the prosthesis thereon. This decision may be made automatically, for example including only the adjacent teeth **A, B,** or the tooth **D** directly opposite the preparation area **P**, or any other appropriate tooth or combination of teeth. A suitable algorithm may be provided that recognizes which entity in **I_{1'}** corresponds to the area **P**, for example by determining the height of each tooth entity, and working out the entity having the shortest height. The height of each tooth may be obtained from the corresponding entity **I_{1'}** by suitable algorithms and routines, as known in the art. The spatial relationship between the entity corresponding to the area **P** and the entities corresponding to the other teeth can then be determined in an automated manner by using simple geometrical rules.

Alternatively, the choice may be made manually, and this may be done, for example, by displaying the scanned and separated teeth entities on a suitable display, such as a computer screen for example, and then marking the desired tooth or teeth by means of a cursor or the like. It may be preferable to manually perform this selection, as it would be important not to include certain teeth with particular visual defects or irregularities, e.g. cavities, parts that require restorations, spots that need to be removed etc. that it is not wished to repeat in the prosthesis.

The data sets **I_{P1'}** corresponding to the chosen teeth are then analysed in turn, and in step **1460** a colour map for the prosthesis is provided based on the colour data provided from data base **I_{P1'}**.

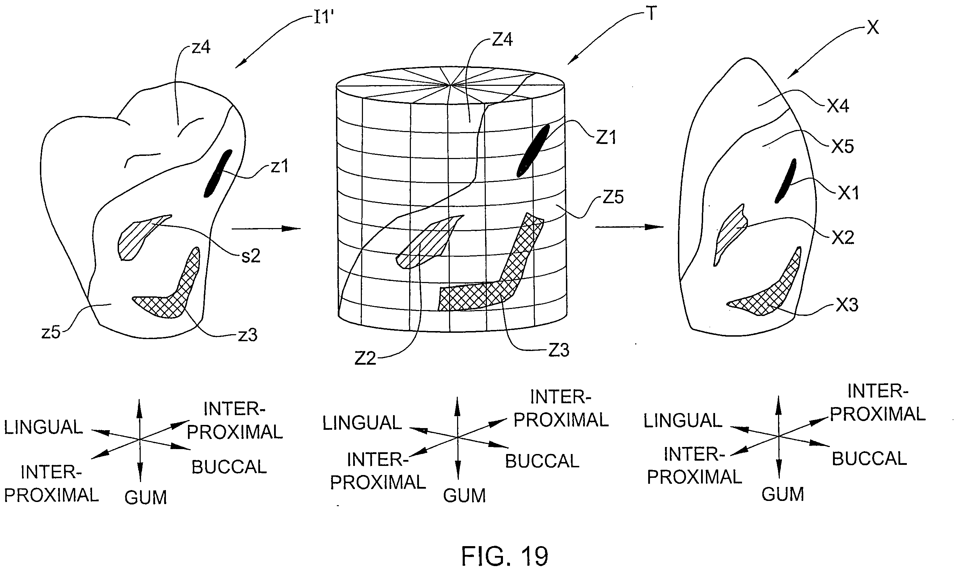

Referring to Fig. 19, for each of the teeth chosen, the appropriate entities are each geometrically transformed to conform to a predetermined geometrical shape, for the sake of example illustrated in this figure as a cylinder, to provide a transformed entity **T.** In the transformation process, the surface geometry of **I_{P1'}** is transformed into the surface geometry of **T**, and the values of the colour parameters c associated with every data point are correspondingly transferred. Effectively, then, the colour attributes of each individual entity **I_{1'},** such as for example features **z1, z2, z3**, and the two differently coloured zones **z4**, **z5** exemplified in Fig. 19, are thus mapped onto the entity **T** into areas **Z1**, **Z2**, **Z3**, **Z4** and **Z5** respectively.

When only one tooth is chosen for basing the shading of the prosthesis on, i.e., corresponding to only a single entity **I_{P1'}**, the transformed entity **T** thus obtained is then transformed again to assume the shape of the prosthesis, providing a prosthesis entity **X**, effectively mapping all the colour features of the entity **I_{P1'}** thereto. The shape of the prosthesis is previously determined by any suitable means, and this does not form part of the present invention. The entity **X** thus comprises the surface geometry of the prosthesis, and the shading information with respect to this geometry, including features **x1-x5** transferred from features **Z1-Z5** respectively, that will provide a similar appearance to that of the tooth on which the colour was modelled. In such a case, the intermediate step of transforming **I_{P1'}** to **T** may be dispensed with, and thus the entity **I_{P1'}** may be transformed directly into the form of the crown prosthesis, thereby providing the values for the colour c thereof.

Nevertheless, the inclusion of intermediate entity **T** may be useful. Optionally, the tooth may be divided into three general zones: a gingival zone close to the gums, an intermediate body zone, and an incisal zone comprising the cusp of the tooth. The colours can be mapped into each of the zones independently, and then smoothed out between the zones to avoid sharp discontinuities in colour.

When a number of teeth are chosen to serve as the basis for shading the prosthesis, the entities **I_{1'}** corresponding to each of these teeth is transformed to a corresponding entity **T'**. Then, the colour data of the entities **T'** are combined to provide a composite entity **T** of the same shape but having composite shading information obtained from all the entities **I_{1'}**. For example the colour value at every geometrical point for all the entities **T'** could be averaged. Alternatively, a weighted average of the colours could be provided, wherein more weight is given to teeth that are closest in location and/or function than to other teeth. Again, when such a combination of the colour information is effected, it is important to ensure that the various entities **T'** are aligned with respect to the individual interproximal, buccal and lingual sides. The composite entity **T** is then transformed geometrically to conform to the shape of the prosthesis to provide entity **X** as described before, but wherein the composite colour values for parameter c are now transferred to the geometrical shape of the entity.

In the above example, the prosthesis has been exemplified as a crown. Nevertheless, the method may be applied to a bridge prosthesis, filing, restoration, or tooth transplant in a similar manner to that described, *mutatis mutandis.*

While the above methods have been described as operations carried out on discrete data points, it is clear that the method of the invention is applicable to similar operations being carried out on new data points suitably interpolated with respect to the original data points, *mutatis mutandis.* Furthermore, it is also possible to carry out the method, of the invention when the numerical entity is structured in a different manner to that described herein, mutatis mutandis. For example, rather than being described as a series of discrete data points on a surface, the surface of the intra-oral cavity could be described as a series of segments, by suitable formulae, or by any other geometric modelling method.

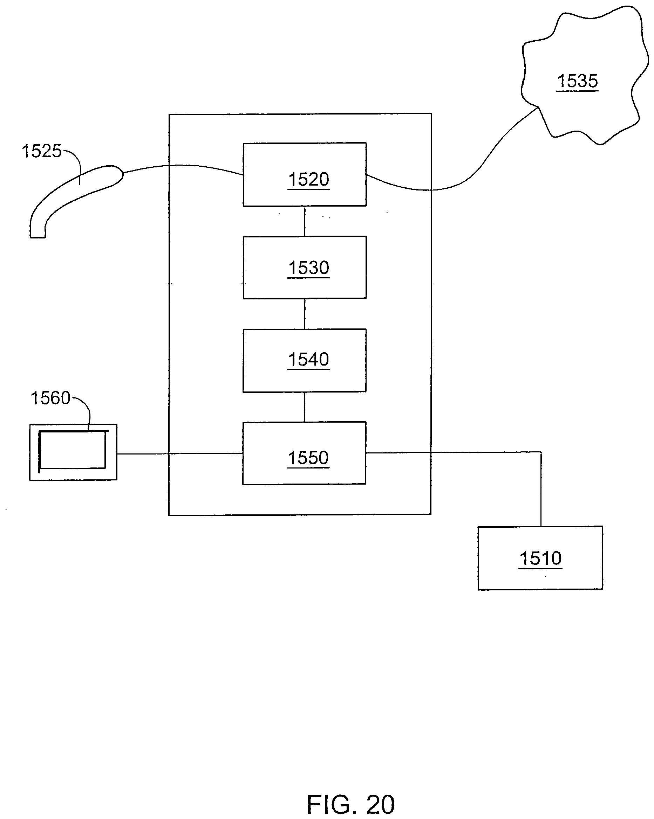

Fig. 20 is a block diagram of a system **1500.** System **1500** presents a software/hardware utility connectable to a virtual treatment system **1510,** for example. System **1500** comprises such main elements as follows:
- an input utility **1520** for receiving data indicative of three dimensional colour data of the intraoral cavity, obtained directly using scanner **1525** (for example the device 100 described herein in reference to Figs. 1 to 13) or obtained elsewhere and transmitted to the input facility via any communication medium **1535,** including for example the internet, an intranet, and so on;
- a memory utility **1530** for storing the data;
- a processor utility **1540** for manipulating said first data set to provide desired data therefrom, particularly using any algorithm according to the present method; and
- output utility **1550.**

System **1500** can be connected to a display **1560** or a printer (not shown) for visually presenting the manipulated entities. System **1500** can be also connectable to an additional utility such as a virtual treatment system **1510.**

In another aspect of the present invention, a computer readable medium is provided that embodies in a tangible manner a program executable for providing data useful in procedures associated with the oral cavity. The computer readable medium comprises:
(a) a first set of data representative of the three dimensional surface geometry and colour of at least part of the intra oral cavity;
(b) means for manipulating said first data set to provide desired data therefrom.

The medium may comprise, for example, optical discs, magnetic discs, magnetic tapes, and so on.

In the method claims that follow, alphabetic characters and Roman numerals used to designate claim steps are provided for convenience only and do not imply any particular order of performing the steps.

Finally, it should be noted that the word "comprising" as used throughout the appended claims is to be interpreted to mean "including but not limited to".

## Claims

1. Computer-implemented method of providing a finish line for a preparation area in an oral cavity (26), the finish line being a circumferential junction between an upper prepared portion of a tooth and a lower unprepared portion of the tooth, comprising:
providing at least one numerical entity (I) representative of the three-dimensional surface geometry and colour of at least part of the intra-oral cavity; and
manipulating said entity to provide desired data therefrom,
wherein said numerical entity comprises surface geometry in form of spatial coordinates (x, y, z) and colour data (c) associated with said part of the intra-oral cavity, wherein said method is useful for differentiating between two tissues and comprises further:
(a) comparing the colour data for each pair of spatially adjacent data points in said numerical entity;
(b) if the colour data for said pair of data points are substantially different one from the other, providing one said data point of said pair of data points having colour data associated with a hard tissue; and
(c) applying a search algorithm to the numerical entity for identifying said finish line in said numerical entity, wherein said application of said algorithm is initially applied to a part of said numerical entity corresponding to the said provided data points of step (b) as a starting point.

2. Computer-implemented method according to claim 1, wherein said colour data includes actual or perceived visual characteristics including hue, chroma, value, translucency, reflectance.

3. Computer-implemented method according to claim 1, particularly useful for differentiating a first tissue from a second tissue, wherein said first tissue comprises substantially different colour characteristics from those of said second tissue, comprising:
separating said surface geometry and colour data into at least two tissue data sets, wherein
a first said tissue data set comprises surface geometry and colour data, wherein said colour data thereof is correlated with a colour representative of said first tissue; and
a second said tissue data set comprises surface geometry and colour data, wherein said colour data thereof is correlated with a colour representative of said second tissue.

4. Computer-implemented method according to claim 3, wherein said first tissue and said second tissues comprise hard tissues and soft tissues, respectively, of the intraoral cavity.

5. Computer-implemented method according to claim 4, wherein optionally said first tissue comprises teeth, wherein further optionally said second tissue includes at least one of gums, tongue, cheeks and lips, and wherein further optionally said first tissue data set corresponds to a plurality of teeth of said intraoral cavity.

6. Computer-implemented method according to claim 4, wherein said first tissue data set corresponds to a plurality of teeth of said intraoral cavity, and wherein said first tissue data set is divided into a plurality of sub data sets, wherein each said sub data set correspond to a different said tooth.

7. Computer-implemented method according to claim 6, optionally further comprising the step of manipulating said sub data sets in a manner simulating an orthodontic treatment on said teeth, and further optionally further comprising the step of displaying said sub-data sets as images corresponding to individual teeth.

8. A computer readable medium that embodies in a tangible manner a program executable for providing a finish line for a preparation in an oral cavity in accordance with a method according to one of the preceding claims.

9. A computer-readable medium according to claim 8, wherein the medium comprises at least one of optical discs, magnetic discs, magnetic tapes, and the like.

## Patentansprüche

1. Computerimplementiertes Verfahren zum Bereitstellen einer Endbearbeitungslinie für einen Präparationsbereich in einer Mundhöhle (26), wobei die Endbearbeitungslinie eine Umfangsverbindung zwischen einem oberen präparierten Abschnitt eines Zahns und einem unteren unpräparierten Abschnitt des Zahns ist, umfassend:
Bereitstellen von mindestens einer numerischen Einheit (I), welche die dreidimensionale Oberflächengeometrie und Farbe von mindestens einem Teil der Mundhöhle repräsentiert; und
Manipulieren der Einheit dazu, die erwünschten Daten daraus bereitzustellen,
wobei die numerische Einheit Oberflächengeometrie in Form von räumlichen Koordinaten (x, y, z) und Farbdaten (c) umfasst, die dem Teil der Mundhöhle zugeordnet sind, wobei das Verfahren zum Differenzieren zwischen zwei Geweben verwendbar ist und des Weiteren umfasst:
(a) Vergleichen der Farbdaten für jedes Paar von räumlich benachbarten Datenpunkten in der numerischen Einheit;
(b) wenn die Farbdaten für das Paar von Datenpunkten im Wesentlichen unterschiedlich voneinander sind, Bereitstellen des einen Datenpunkts aus dem Paar von Datenpunkten, der Farbdaten aufweist, die Hartgewebe zugeordnet sind; und
(c) Anwenden eines Suchalgorithmus auf die numerische Einheit zum Identifizieren der Endbearbeitungslinie in der numerischen Einheit, wobei das Anwenden des Algorithmus anfänglich auf einen Teil der numerischen Einheit, der den bereitgestellten Datenpunkten aus Schritt (b) entspricht, als ein Anfangspunkt angewendet wird.

2. Computerimplementiertes Verfahren nach Anspruch 1, wobei die Farbdaten tatsächliche oder wahrgenommene visuelle Merkmale einschließlich Farbton, Chroma, Wert, Transluzenz, Reflexionsgrad beinhalten.

3. Computerimplementiertes Verfahren nach Anspruch 1, das insbesondere zum Unterscheiden eines ersten Gewebes von einem zweiten Gewebes verwendet werden kann, wobei das erste Gewebe im Wesentlichen unterschiedliche Farbmerkmale von denjenigen des zweiten Gewebes umfasst, umfassend:
Aufteilen der Oberflächengeometrie und der Farbdaten in mindestens zwei Gewebedatensätze, wobei
ein erster Gewebedatensatz Oberflächengeometrie und Farbdaten umfasst, wobei die Farbdaten davon mit einer Farbe korreliert sind, die das erste Gewebe repräsentiert;
und
ein zweiter Gewebedatensatz Oberflächengeometrie und Farbdaten umfasst, wobei die Farbdaten davon mit einer Farbe korreliert sind, die das zweite Gewebe repräsentiert.

4. Computerimplementiertes Verfahren nach Anspruch 3, wobei das erste Gewebe und das zweite Gewebe Hartgewebe bzw. Weichgewebe in der Mundhöhle umfassen.

5. Computerimplementiertes Verfahren nach Anspruch 4, wobei optional das erste Gewebe Zähne umfasst, wobei weiterhin optional das zweite Gewebe mindestens eines aus Gaumen, Zunge, Wangen und Lippen beinhaltet, und wobei weiterhin optional der erste Gewebedatensatz einer Vielzahl von Zähnen in der Mundhöhle entspricht.

6. Computerimplementiertes Verfahren nach Anspruch 4, wobei der erste Gewebedatensatz einer Vielzahl von Zähnen in der Mundhöhle entspricht, und wobei der erste Gewebedatensatz in eine Vielzahl von Teildatensätze aufgeteilt ist, wobei jeder Teildatensatz einem anderen Zahn entspricht.

7. Computerimplementiertes Verfahren nach Anspruch 6, optional des Weiteren umfassend den Schritt zum Manipulieren der Teildatensätze auf eine Weise, die eine orthodontische Behandlung an diesem Zahn simuliert, und des Weiteren optional umfassend den Schritt zum Anzeigen der Teildatensätze als Bilder, die einzelnen Zellen entsprechen.

8. Computerlesbares Medium, das in einer greifbaren Weise ein Programm verkörpert, das ausführbar ist, um eine Endbearbeitungslinie für eine Präparation in einer Mundhöhle entsprechend einem Verfahren nach einem der vorhergehenden Ansprüche bereitzustellen.

9. Computerlesbares Medium nach Anspruch 8, wobei das Medium mindestens eines aus optischen Laufwerken, Plattenspeichern, Magnetbändern und dergleichen umfasst.

## Revendications

1. Procédé informatique de fourniture d'une ligne finie pour une zone de préparation dans une cavité buccale (26), la ligne finie étant une jonction circonférentielle entre une partie supérieure préparée d'une dent et une partie inférieure non préparée de la dent, comprenant :
la fourniture d'au moins une entité numérique (I) représentative de la géométrie de surface tridimensionnelle et de la couleur d'au moins une partie de la cavité intrabuccale ; et
la manipulation de ladite entité pour fournir les données souhaitées à partir de celles-ci,
où ladite entité numérique comprend la géométrie de surface sous forme de coordonnées spatiales (x, y, z) et de données de couleur (c) associées à ladite partie de la cavité intrabuccale, où ledit procédé est utile pour la différenciation entre deux tissus et comprend en outre :
(a) la comparaison des données de couleur pour chaque paire de points de données spatialement adjacents dans ladite entité numérique ;
(b) si les données de couleur pour ladite paire de points de données sont sensiblement différentes l'une de l'autre, la fourniture de l'un dudit point de données de ladite paire de points de données ayant des données de couleur associées à un tissu dur ; et
(c) l'application d'un algorithme de recherche à l'entité numérique pour identifier ladite ligne finie dans ladite entité numérique, ladite application dudit algorithme étant initialement appliquée à une partie de ladite entité numérique correspondant auxdits points de données fournis de l'étape (b) comme point de départ.

2. Procédé informatique selon la revendication 1, lesdites données de couleur comprenant des caractéristiques visuelles réelles ou perçues comprenant la teinte, la saturation, la valeur, la translucidité, la réflectance.

3. Procédé informatique selon la revendication 1, particulièrement utile pour la différenciation d'un premier tissu vis-à-vis d'un second tissu, ledit premier tissu comprenant des caractéristiques de couleur sensiblement différentes de celles dudit second tissu, comprenant :
la séparation de ladite géométrie de surface et des données de couleur en au moins deux ensembles de données de tissu, où
un premier ensemble desdites données de tissu comprenant les données de géométrie de surface et de couleur, lesdites données de couleur de celui-ci étant corrélées à une couleur représentative dudit premier tissu ; et
un second ensemble desdites données de tissu comprenant des données de géométrie de surface et de couleur, lesdites données de couleur de celui-ci étant corrélées à une couleur représentative dudit second tissu.

4. Procédé informatique selon la revendication 3, dans lequel ledit premier tissu et ledit second tissu comprennent respectivement des tissus durs et des tissus mous, de la cavité intrabuccale.

5. Procédé informatique selon la revendication 4, dans lequel éventuellement ledit premier tissu comprend des dents, où en outre éventuellement ledit second tissu comprend au moins l'une parmi les gencives, la langue, les joues et les lèvres, et où en outre éventuellement ledit premier ensemble de données de tissu correspond à une pluralité de dents de ladite cavité intrabuccale.

6. Procédé informatique selon la revendication 4, dans lequel ledit premier ensemble de données de tissu correspond à une pluralité de dents de ladite cavité intrabuccale, et où ledit premier ensemble de données de tissu est divisé en une pluralité de sous-ensembles de données, où chacun dudit sous-ensemble de données correspond à une dite dent différente.

7. Procédé informatique selon la revendication 6, comprenant en outre éventuellement l'étape de manipulation desdits sous-ensembles de données d'une manière simulant un traitement orthodontique sur lesdites dents, et comprenant en outre éventuellement de plus l'étape d'affichage desdits sous-ensembles de données comme images correspondant à des dents individuelles.

8. Milieu informatique qui concrétise d'une manière tangible un programme exécutable de fourniture d'une ligne finie pour une préparation dans une cavité buccale selon un procédé selon l'une des revendications précédentes.

9. Milieu informatique selon la revendication 8, le milieu comprenant au moins l'un parmi des disques optiques, des disques magnétiques, des bandes magnétiques, et similaires.
